# FASCICULE DE BREVET EUROPEEN

(11) **EP 0 745 584 B1**
(45) Date de publication et mention de la délivrance du brevet: **05.01.2000**
(21) Numéro de dépôt: 96401159.7
(22) Date de dépôt: 30.05.1996
(51) Int. Cl.: C07C 235/34, C07C 235/36, C07D 209/18, C07D 333/24, C07D 333/32, C07D 307/79, C07D 307/92, C07D 311/92, A61K 31/165, A61K 31/40, A61K 31/38, A61K 31/34

(54) **Composés arylalkyl(thio) carboxamides ayant une affinité pour des récepteurs mélatoninergiques, leurs procédés de préparation et les compositions pharmaceutiques qui les contiennent**
Arylalkyl(thio)carboxamidverbindungen mit Affinität für Melatoninrezeptoren, Verfahren zu ihrer Herstellung und sie enthaltende pharmazeutische Zusammensetzungen
Arylalkyl(thio) carboxamides compounds with affinity for melatonine receptors, processes for their preparation and pharmaceutical compositions containing them

(30) Priorité: 31.05.1995 FR 9506435
(43) Date de publication de la demande: 04.12.1996
(73) Titulaire: ADIR ET COMPAGNIE, 92415 Courbevoie Cédex (FR)
(72) Inventeur: Lesieur, Isabelle, 59147 Gondecourt (FR); Depreux, Patrick, 59280 Armentieres (FR); Leclerc, Véronique, 51000 Lille (FR); Delagrange, Philippe, 92130 Issy les Moulineaux (FR); Renard, Pierre, 78000 Versailles (FR); Guardiola Lemaitre, Béatrice, 92210 Saint Cloud (FR)

(56) Documents cités:
- EP-A- 0 447 285
- EP-A- 0 527 687
- EP-A- 0 530 087
- EP-A- 0 719 760
- JOURNAL OF MEDICINAL CHEMISTRY, vol. 13, no. 6, WASHINGTON US, pages 1205-1208, XP002011167 E. CAMPAIGNE ET AL.: "Benzo[b]thiophene derivatives. XVI. The sulfur isosteres of melatonin, bufotenine, 5-hydroxytryptophan, and related structures"

## Description

L'invention concerne de nouveaux composés arylalkyl(thio)carboxamides, leurs procédés de préparation et les compositions pharmaceutiques qui les contiennent.

L'invention décrit de nouveaux composés arylalkyl(thio)carboxamides qui s'avèrent être de puissants ligands des récepteurs mélatoninergiques.

On connait dans l'art antérieur des dérivés (indol-3yl)alkyl(thio)amides qui sont utilisés comme intermédiaires des synthèses de dérivés hétérocycliques thioisomünchnone (Padwa A. et al. Synthesis 1994, n°9, pp 993-1004) ou comme produits de la réaction de Ritter (Kost A.N. et al. Vest. Mosk. Univ. Khim., 1975, 16, pp 22-6).

On connait également le N-méthyl-3-(4-méthoxy-napht-1-yl)propionamide et le N-tertbutyl-3-carboxy-4-(napht-1-yl)butyramide utiles comme intermédiaires de synthèse pour préparer respectivement une benzocyclohexanedione lactone (Taylor E.C. et al. J. Am. Chem. Soc., 1981, 103, pp 6856-6863) et un inhibiteur de la rénine (EP 427939) ainsi qu'un dérivé naphth-2-ylacétamide utile pour la préparation de dérivés d'acide hydroxamique (Hoffman R.V. et al. J. Org. Chem., 1992, 57, pp 5700-5707).

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720), et sur le diabète (Clinical endocrinolgy, 1986, 24, pp 359-364).

Des composés permettant d'agir sur le système mélatoninergique sont donc pour le clinicien d'excellents médicaments pour le traitement des pathologies liées aux troubles du système mélatoninergique, et notamment celles mentionnées précédemment. L'invention concerne les composés de formule (I) : 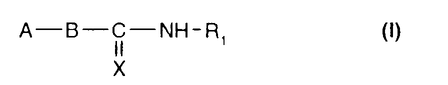 dans laquelle :
- A représente un groupement de formule : 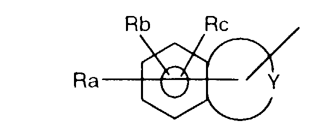
dans laquelle :
- Y forme avec le noyau benzo qui le porte un cycle choisi parmi naphtalène, dihydronaphtalène, tétrahydronaphtalène, benzofurane, 2,3-dihydrobenzofurane, benzothiophène, 2,3-dihydrobenzothiophène, indole, indoline, 2H-indène et indane ;
- Ra, Rb et Rc, chacun indépendamment l'un de l'autre, représentent un hydrogène ou un radical choisi parmi halogène, hydroxy, -Rd et -O-Rd ; avec Rd étant choisi parmi alkyle, alkyle substitué par un ou plusieurs halogènes, alcényle, alcynyle, cycloalkyle, cycloalkylalkyle, aryle, arylalkyle, aryle substitué, arylalkyle substitué ;
   Ra pouvant également former avec Rb et les 2 atomes de carbone adjacents qui les portent, un cycle A₁ choisi parmi furane, dihydrofurane, pyrane et dihydropyrane, A₁ étant éventuellement substitué par un ou plusieurs radicaux ou groupements choisis parmi hydroxy, alkyle, alkoxy et oxo ;
- B représente une chaîne alkylène de 2 à 6 atomes de carbone non substituée ou substituée par un ou plusieurs radicaux choisis parmi alkyle, alkoxy, hydroxy, carboxy, alkoxycarbonyle, carboxyalkyle et alkoxycarbonylalkyle étant entendu que B peut également représenter une chaine méthylène lorsque Y forme avec le noyau benzo qui le porte un naphtalène, un dihydronaphtalène ou un tétrahydronaphtalène et que Ra, Rb et Rc ne représentent pas simultanément des hydrogènes ;
   avec la réserve que Ra, Rb et Rc ne peuvent représenter simultanément des hydrogènes ou un hydrogène et 2 radicaux méthyle ou 2 hydrogènes et un radical méthyle, lorsque Y forme avec le noyau benzo auquel il est lié un indole,
   et que le composé de formule (I) ne peut pas être le N-méthyl-3-(4-méthoxy-napht-1-yl)propionamide ni le N-tertbutyl-3-carboxy-4-(napht-1-yl)butyramide,
- X représente un oxygène ou un soufre ;
- et R₁ représente un radical choisi parmi alkyle, alkyle substitué par un ou plusieurs halogènes, alcényle, alcynyle, cycloalkyle et cycloalkylalkyle,
étant entendu que :
lorsque Y forme avec le noyau benzo qui le porte un cycle naphtalène, B représente une chaîne alkylène de 2 à 6 atomes de carbone, X représente un atome d'oxygène, 2 des groupements Ra, Rb ou Rc représentent simultanément un atome d'hydrogène et le troisième représente un groupement hydroxy ou alkoxy (C₁-C₆) linéaire ou ramifié (Ra est dans ce cas un substituant de la partie benzo),
alors R₁ ne peut représenter un groupement alkyle ou alkyle substitué par un groupement cycloalkyle,
étant entendu que :
- les termes "alkyle" et "alkoxy" désignent des groupements linéaires ou ramifiés contenant de 1 à 6 atomes de carbone,
- les termes "alcényle" et "alcynyle" désignent des groupements insaturés linéaires ou ramifiés contenant de 2 à 6 atomes de carbone,
- le terme "cycloalkyle" désigne un groupement de 3 à 8 atomes de carbone,
- le terme "aryle" désigne un radical phényle, naphtyle ou pyridyle,
- le terme "substitué" affecté aux expressions "aryle" et "arylalkyle" signifie que ces groupements peuvent être substitués par un ou plusieurs radicaux choisis parmi halogène, alkyle, alkoxy, hydroxy et alkyle substitué par un ou plusieurs halogènes ;
leurs énantiomères et diastéréoisomères,
et leurs sels d'addition à un acide ou une base pharmaceutiquement acceptable.

L'invention concerne particulièrement les composés de formule (I) dans laquelle, pris séparément ou ensemble :
- A représente un naphtyle,
- A représente un naphtyle substitué,
- A représente un naphtyle partiellement hydrogéné, par exemple un 1,2-dihydronaphtyle substitué ou 1,2,3,4-tétrahydronaphtyl substitué,
- A représente un naphtyle substitué partiellement hydrogéné, par exemple un 1,2-dihydronaphtyle substitué ou 1,2,3,4-tétrahydronaphtyl substitué,
- A représente un benzofuryle,
- A représente un benzofuryle substitué,
- A représente un benzothiényle,
- A représente un benzothiényle substitué,
- A représente un indolyle,
- A représente un indolyle substitué,
- A est substitué par un radical alkoxy,
- A est substitué par un halogène,
- A est substitué par un alkyle,
- B représente une chaîne méthylène,
- B représente une chaîne éthylène,
- B représente une chaîne -(CH₂)₃-,
- X représente un soufre,
- X représente un oxygène,
- R₁ représente un alkyle,
- R₁ représente un alkyle substitué par un ou plusieurs halogènes,
- R₁ représente un cycloalkyle,
- et R₁ représente un cycloalkyalkyle.

Par exemple, l'invention concerne les composés particuliers de formule (I) répondant aux formules (1) à (12) suivantes: 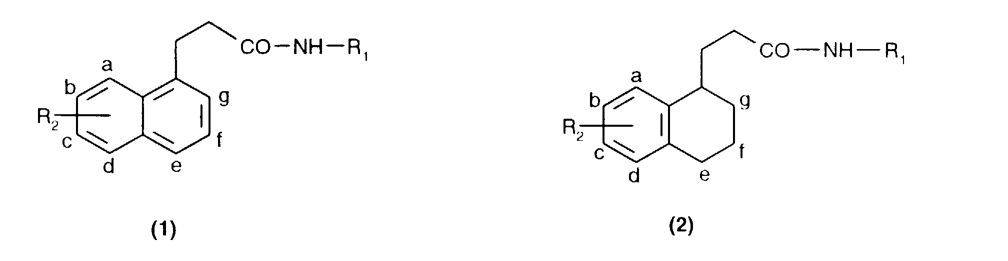 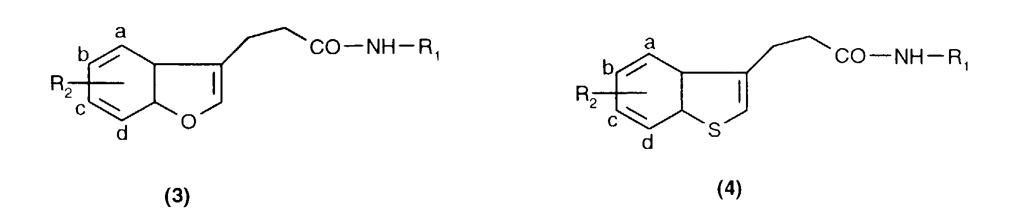 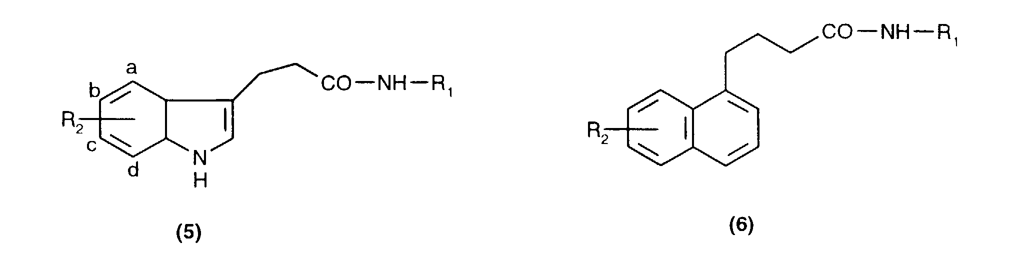 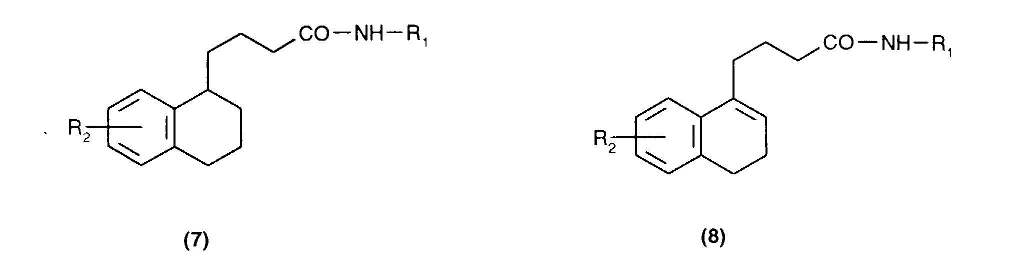 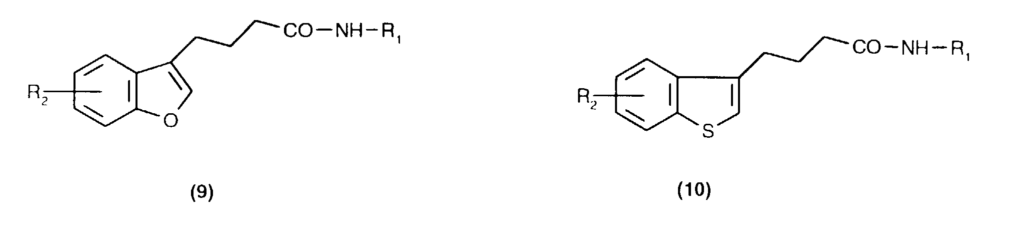 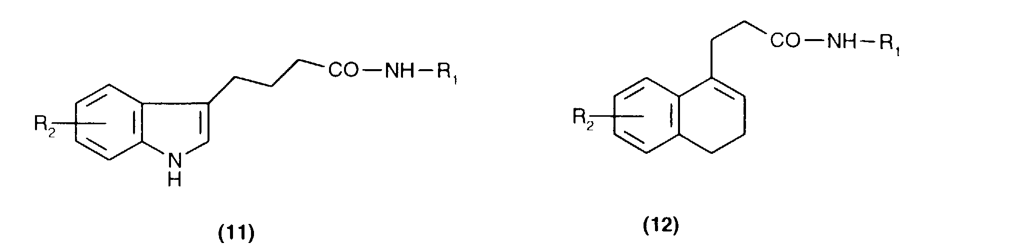 dans lesquelles R₂ représente un radical choisi parmi halogène, hydroxy, -Rd et -O-Rd avec Rd tel que défini dans la formule (I).

Par exemple R₂ représente un radical choisi parmi halogène, alkyle et alkoxy.

L'invention conceme plus particulièrement les composés de formule (I) dans laquelle le radical R₂ est en position b du naphtyle ou du tétrahydronaphtyle et les composés de formule (I) dans laquelle R₂ est en position b du cycle benzofuryle, benzothiophène ou indolyle.

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle et hexyle et les isomères de squelette des radicaux pentyles et hexyles,
les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy et hexyloxy et les isomères de squelette des radicaux pentyloxy et hexyloxy,
les halogènes présents dans la formule (I) peuvent être choisis parmi le brome, le chlore, le fluor et l'iode,
les cycloalkyles présents dans la formule (I) peuvent être choisis parmi cyclopropyle, cyclobutyle, cyclopentyle, cyclohexyle, cycloheptyle et cyclooctyle,
et les groupements alkylènes présents dans la formule (I) peuvent être choisis parmi méthylène, éthylène, triméthylène, tétraméthylène, pentaméthylène et hexaméthylène.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

La présente invention a également pour objet l'utilisation pour l'obtention de compositions pharmaceutiques destinées au traitement des troubles du système mélatoninergique et des troubles liés au système mélatoninergique et plus particulièrement au traitement des dépressions saisonnières, des troubles du sommeil, des pathologies cardiovasculaires, des insomnies et fatigues dues aux décalages horaires, des troubles de l'appétit et de l'obésité, des composés de formule (I_{A}): 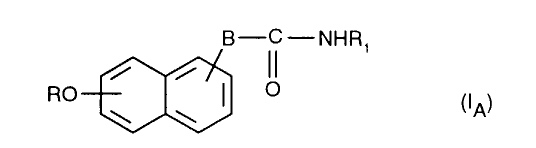 dans laquelle :
- R représente un atome d'hydrogène ou un groupement alkyle (C₁-C₆) linéaire ou ramifié,
- B représente une chaîne alkylène de 2 à 6 atomes de carbone,
- R₁ représente un groupement alkyle (C₁-C₆) linéaire ou ramifié non substitué ou substitué par un ou plusieurs groupements cycloalkyle (C₃-C₈),
leurs énantiomères et diastéréoisomères,
ainsi que leurs sels d'addition à un acide ou à une base pharmaceutiquement acceptable.

L'invention concerne également un procédé de préparation des composés de formule (I) caractérisé en ce que on fait réagir un acide de formule (II):

A―B―COOH **(II)**

dans laquelle A et B sont tels que définis dans la formule (I),
avec une amine de formule (III) :

H₂N―R₁ **(III)**

dans laquelle R₁ est tel que défini dans la formule (I),
pour obtenir le composé de formule (I/a) : 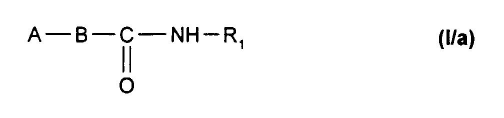 dans laquelle A, B et R₁ sont tels que définis précédemment,
composé de formule (I/a) qui, traité par un réactif de thionation, par exemple le réactif de Lawesson, donne le composé de formule (I/b) : 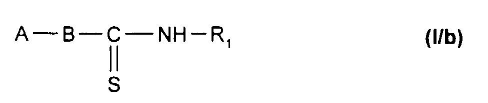 dans laquelle A, B et R₁ sont tels que définis précédemment,
les composés de formule (I/a) et (I/b) formant l'ensemble des composés de formule (I) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

L'invention concerne également le procédé de préparation des composés de formule (I/c) cas particulier des composés de formule (I) : 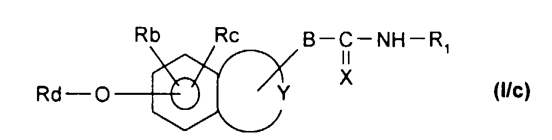 dans laquelle B, X, Y, R₁, R_{b}, R_{c} et R_{d} sont tels que définis dans la formule (I) caractérisé en ce que l'on greffe le radical R_{d} sur un composé de formule (I/d) : 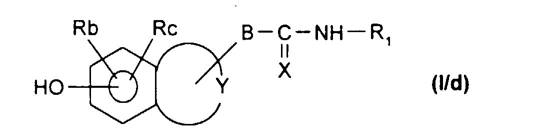 dans laquelle B, X, Y, R₁, R_{b} et R_{c} sont tels que définis précédemment.
composés de formule (I/c) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

Le greffage du radical R_{d} peut par exemple être réalisé par l'intermédiaire d'un composé de formule (IV) :

Rd―W (IV)

dans laquelle R_{d} est tel que défini dans la formule (I) et W représente un halogène ou un groupement partant.

Les composés de formule (I/d) : 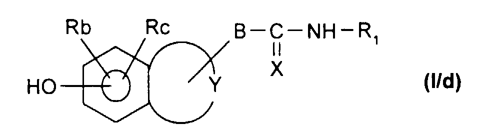 dans laquelle B, X, Y, R₁, R_{b} et R_{c} sont tels que définis dans la formule (I) sont accessibles à l'homme du métier par déalkylation d'un composé de formule (I/e) : 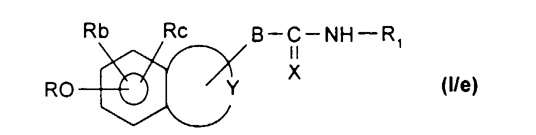 dans laquelle B, X, Y, R₁, R_{b} et R_{c} sont tels que définis précédemment et R représente un (C₁-C₆)alkyle, par exemple un méthyle.

Par exemple, la déalkylation du composé de formule (I/e) peut être réalisé grâce au BBr₃ ou à un mélange AlX'₃, R'-SH dans lequel X' est un halogène et R' est un (C₁-C₆)alkyl.

L'invention concerne également le procédé de préparation des composés de formule (I/f) : 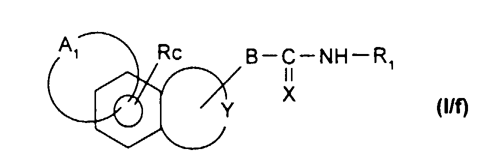 dans laquelle B, X, Y, R₁, R_{c} et A₁ sont tels que définis dans la formule (I) par cyclisation d'un composé de formule (I/g) : 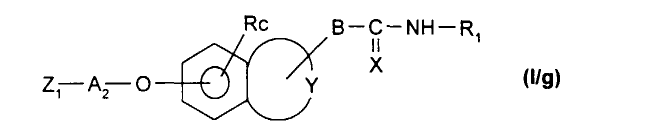 dans laquelle B, X, Y, R₁ et R_{c} sont tels que définis précédemment, A₂ représente une chaîne choisie parmi (C₂-C₃)alkylène et (C₂-C₃)alcénylène , A₂ étant non substitué ou substitué par un ou plusieurs groupements choisis parmi hydroxy, alkyl, alkoxy et oxo, et Z₁ étant une fonction réactive ,
composés de formule (I/f) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

L'invention concerne également le procédé de préparation d'un composé de formule (I/h) : 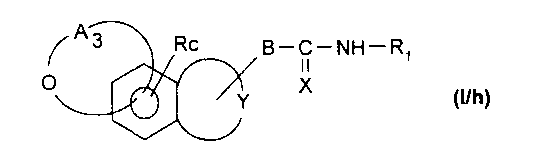 dans laquelle B, X, Y, R₁, et R_{c} sont tels que définis dans la formule (I) et A₃ représente une chaîne (C₂-C₃)alkylène non substituée ou substituée par un radical (C₁-C₆)alkyle caractérisé en ce qu'on cyclise un composé de formule (I/i) : 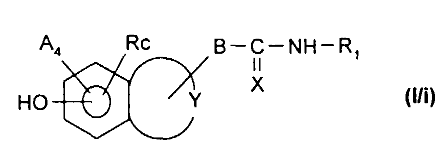 dans laquelle B, X, Y, R₁, et R_{c} sont tels que définis précédemment et A₄ représente un radical (C₂-C₃) alcényl non substitué ou substitué par un radical (C₁-C₆)alkyl,
composés de formule (I/h) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

L'invention concerne également le procédé de préparation des composés de formule (I/j) : 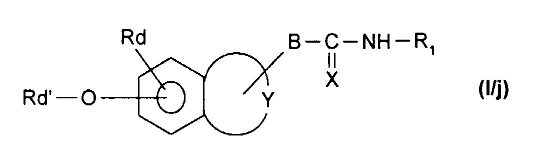 dans laquelle Rd, Y, X, B, et R₁ sont tels que définis dans la formule (I) et Rd' peut prendre les même valeurs que Rd tel que défini précédemment,
caractérisé en ce que on greffe le radical Rd tel que défini précédemment sur la fonction hydroxy d'un composé de formule (I/k) : 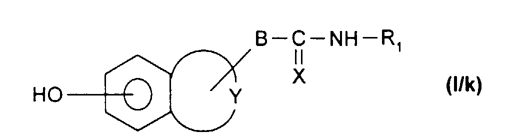 dans laquelle Y, B, X, et R₁ sont tels que définis précédemment pour obtenir un composé de formule (I/ℓ) : 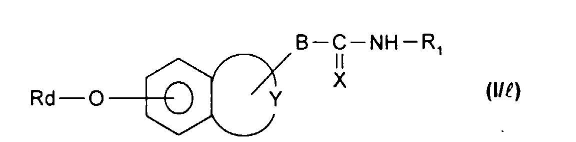 dans laquelle Rd, Y, B, X et R₁ sont tels que définis précédemment,
composé de formule (I/ℓ) qui est ensuite soumis à une réaction de transposition pour obtenir le composé de formule (I/m) : 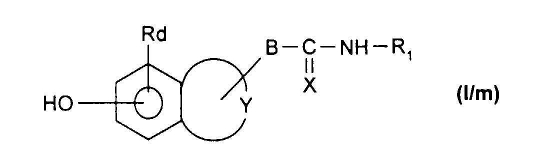 dans laquelle Rd, Y, B, X et R₁ sont tels que définis précédemment,
la fonction hydroxy étant de nouveau greffée par un radical Rd' tel que défini précédemment pour obtenir un composé de formule (I/j) : 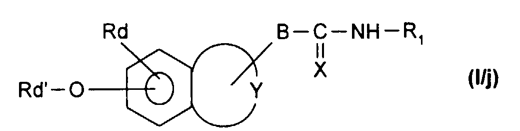 dans laquelle Rd, d', Y, B, X et R₁ sont tels que définis précédemment,
les composés de formule (I/j) qui peuvent être , si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique et des troubles liés au système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, qui permettent d'établir que les composés de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, du psoriasis, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans le traitement des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement entre 1 à 100 mg, par exemple entre 1 à 10 mg.

Les exemples suivants illustrent l'invention, mais ne la limitent en aucune façon.

### PREPARATION 1 : ACIDE 3-(7-METHOXY NAPHT-1-YL)-PROPANOIQUE

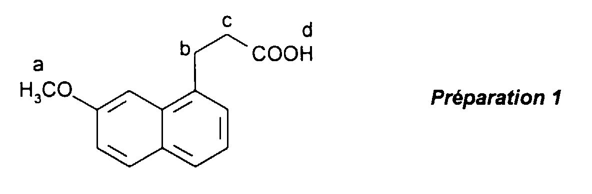

| **Réactifs :** | |
|---|---|
| 3-(7-méthoxy napht-1-yl)propionitrile | 1 g (4,7 x 10⁻³ mole) |
| Solution aqueuse de soude 6N | 10 cm³ (6 x 10⁻² mole) |
| Méthanol | 10 cm³ |

### Mode opératoire :

Dissoudre le 3-(7-méthoxy napht-1-yl) propionitrile dans 10 cm³ de méthanol dans une fiole de 100 cm³. Ajouter la solution aqueuse de soude et porter le milieu à reflux pendant une nuit. Laisser refroidir et acidifier le milieu par une solution aqueuse de HCI 6N puis filtrer le précipité formé. Le recristalliser.

| **Caractéristiques :** | |
|---|---|
| Masse molaire | 230,26 g/mole pour C₁₄H₁₄O₃ |
| Aspect | solide blanc |
| Température de fusion | 154-155°C |
| Rf = 0,40 | |
| Eluant | Acétone/Toluène/Cyclohexane (ci-après ATC) (4/4/2) |
| Rendement | 90 % |
| Solvant de recristallisation | toluène |

| **Analyse spectroscopique dans l'infrarouge :** | |
|---|---|
| 1700 cm⁻¹ | υ CO acide |
| 1620 et 1600 cm⁻¹ | υ C=C |
| 1260 cm⁻¹ | υ CH₃O |

| **Analyse spectroscopique de RMN (80 MHz, DMSO, δ) :** | | | |
|---|---|---|---|
| 2,65 ppm (triplet, 2H) | H_{b} | J_{b-c} | = 7,60 Hz |
| 3,30 ppm (triplet, 2H) | H_{c} | Jc-b | = 7,60 Hz |
| 3,90 ppm (singulet, 3H) | Hₐ | | |
| 7,10-7,40 ppm (massif, 4H) | H2,3,6,8 | | |
| 7,60-7,95 ppm (massif, 2H) | H4,5 | | |
| 12,20 ppm (signal, 1H) | H_{d} échangeable dans D₂O | | |

### PREPARATION 2: ACIDE 4-(7-METHOXY-NAPHT-1-YL)-BUTYRIQUE

### STADE A : 3-(7-METHOXY-NAPHT-1-YL)-PROPANOATE DE METHYLE

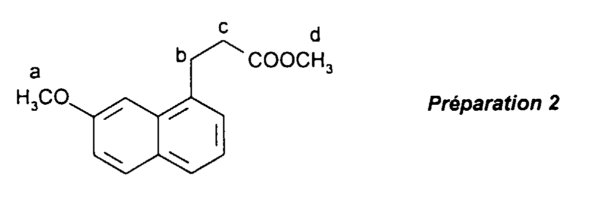

| **Réactifs :** | |
|---|---|
| Acide 3-(7-méthoxy napht-1-yl)propanoique | 0,2 g (0,9 x 10⁻³ mole) |
| Chlorure de thionyle | 0,25 cm³ (3,5 x 10⁻³ mole) |
| Méthanol | 20 cm³ |

### Mode opératoire :

Dans un fiole de 100 cm³ placé dans un bain de glace à - 5°C, dissoudre l'acide dans le méthanol. Ajouter goutte à goutte le chlorure de thionyle et laisser sous agitation pendant 1 h. Evaporer et reprendre le résidu par 10 cm³ d'éther. Laver la phase organique par une solution aqueuse de carbonate de potassium 10 % et par de l'eau.
Sécher sur CaCl₂, filtrer et évaporer.

| **Caractéristiques :** | |
|---|---|
| Masse molaire | 244,29 g/mole pour C₁₅H₁₆O₃ |
| Aspect | huile |
| Rf = 0,67 | |
| Eluant | ATC (4/4/2) |
| Rendement | 89 % |

| **Analyse spectroscopique dans l'infrarouge :** | |
|---|---|
| 3020 - 2800 cm⁻¹ | υ CH |
| 1730 cm⁻¹ | υ CO ester |
| 1620 et 1590 cm⁻¹ | υ C=C |
| 1250 cm⁻¹ | υ CH₃O |

| **Analyse spectroscopique de RMN (300 MHz,CDCl**_{**3**}**, δ) :** | | | |
|---|---|---|---|
| 2,75 ppm (triplet, 2H) | H_{b} | J_{b-c} | = 7,95 Hz |
| 3,34 ppm (triplet, 2H) | H_{c} | J_{c-b} | = 7,95 Hz |
| 3,68 ppm (singulet, 3H) | H_{d} | | |
| 3,90 ppm (singulet, 3H) | Hₐ | | |
| 7,13-7,30 ppm (massif, 4H) | H_{2,3,6,8} | | |
| 7,63 ppm (doublet, 1H) | H₄ | Jₒᵣₜₕₒ | = 7,77 Hz |
| 7,73 ppm (doublet, 1H) | H₅ | Jₒᵣₜₕₒ | = 8,95Hz |

### STADE B : 3-(7-METHOXY NAPHT-1-YL)-PROPAN-1-OL

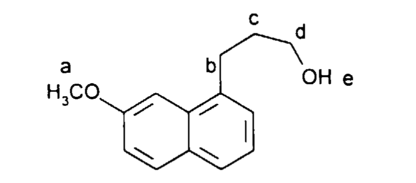

| **Réactifs :** | |
|---|---|
| 3-(7-méthoxy napht-1-yl)-propanoate de méthyle | 0,5 g (2 x 10⁻³ mole) |
| LiAlH₄ | 0,3 g (8 x 10⁻³ mole) |
| Ether anhydre | 10 cm³ |

### Mode opératoire:

Dans un fiole de 50 cm³ placé dans un bain de glace à - 5°C, mettre LiAlH₄ et l'éther puis ajouter goutte à goutte l'ester préalablement dilué dans de l'éther. Laisser agiter pendant 1h et hydrolyser le milieu en le versant sur de l'eau glacée. Filtrer le milieu et l'extraire par de l'éther. Sécher la phase organique, la filtrer et l'évaporer.

| **Caractéristiques :** | |
|---|---|
| Masse molaire | 217,29 g/mole pour C₁₄H₁₆O₂ |
| Aspect | solide blanc |
| Température de fusion | 38-39°C |
| Rf | 0,13 |
| Eluant | chloroforme |
| Rendement | 88 % |
| Solvant de recristallisation | cyclohexane |

| **Analyse spectroscopique dans l'infrarouge :** | |
|---|---|
| 3300 cm⁻¹ | υ OH large bande |
| 3040-2800 cm⁻¹ | υ CH |
| 1620 et 1590 cm⁻¹ | υ C=C |
| 1250 cm⁻¹ | υ CH₃O |

| **Analyse spectroscopique de RMN (300 MHz,CDCl**_{**3**}**, δ) :** | | | |
|---|---|---|---|
| 2,00-2,10 ppm (multiplet, 2H) | H_{c} | | |
| 3,15 ppm (triplet, 2H) | H_{b} | J_{b-c} | = 7,59 Hz |
| 3,75 ppm (triplet, 2H) | H_{d} | J_{d-c} | = 6,24 Hz |
| 3,95 ppm (singulet, 3H) | Hₐ | | |
| 7,13-7,37 ppm (massif, 4H) | H_{2,3,6,8} | | |
| 7,66 ppm (doublet, 1H) | H₄ | Jₒᵣₜₕₒ | = 7,83 Hz |
| 7,76 ppm (doublet, 1H) | H₅ | Jₒᵣₜₕₒ | = 8,92Hz |

### STADE C : MESILATE DU 3-(7-METHOXY-NAPHT-1-YL)PROPAN-1-OL

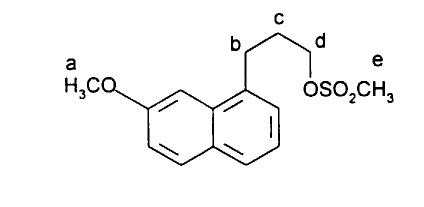

| **Réactifs :** | |
|---|---|
| 3-(7-methoxy-napht-1-yl)propanol | 5,1 g (23,5 x 10⁻³ mole) |
| CH₃SO₂Cl | 3,3 g (28,2 x 10⁻³ mole) |
| NEt₃ | 3,9 cm³ (28,2 x 10⁻³ mole) |
| CH₂Cl₂ | 100 cm³ |

### Mode opératoire :

Dans un ballon de 250 cm³, dissoudre le 3-(7-methoxy-napht-1-yl)propanol dans le CH₂Cl₂. Ajouter la triéthylamine. Placer le ballon dans un bain de glace et de sel (-5°C). Ajouter goutte à goutte le chlorure de mésyle. Laisser sous agitation pendant 2 h. La phase organique est lavée par 3 x 20 cm³ HCI puis par de l'eau jusqu'a neutralité des eaux. Sécher sur CaCl₂, filtrer et évaporer. Recristalliser le solide obtenu.

| **Caractéristiques :** | |
|---|---|
| Masse molaire | 294,37 g/mole pour C₁₅H₁₈O₄S |
| Température de fusion | 52-54°C |
| Rf | 0,71 |
| Eluant | CHCl₃ / MeOH (19/1) |
| Rendement | 71 % |
| Solvant de recristallisation | toluène / cyclohexane (1/3) |

| **Analyse spectroscopique dans l'infrarouge :** | |
|---|---|
| 3040-2820 cm⁻¹ | νCH alkyles |
| 1610 et 1590 cm⁻¹ | νC=C |
| 1330 cm⁻¹ | νSO₂ asym |
| 1260 cm⁻¹ | νOCH₃ |
| 1170 cm⁻¹ | νSO₂ sym |

| **Analyse spectroscopique de RMN (300 MH**_{**z**}**, DMSO, δ)** | | | |
|---|---|---|---|
| 2,08 ppm (multiplet, 2H) | H_{c} | | |
| 3,12 ppm (triplet, 2H) | H_{b} | J_{b-c} | = 7,75 Hz |
| 3,22 ppm (singulet, 3H) | Hₑ | | |
| 3,92 ppm (singulet, 3H) | Hₐ | | |
| 4,32 ppm (triplet, 2H) | H_{d} | J_{d-c} | = 6,20 Hz |
| 7,19 ppm (doublet dédoublé, 1H) | H₆ | Jₒᵣₜₕₒ | = 8,93 Hz |
| | | Jₘₑₜₐ | = 2,38 Hz |
| 7,26-7,37 ppm (massif, 3H) | H_{2,3,8} | | |
| 7,72 ppm (doublet, 1H) | H₄ | Jₒᵣₜₕₒ | = 7,88 Hz |
| 7,85 ppm (doublet, 1H) | H₅ | Jₒᵣₜₕₒ | = 8,93 Hz |

### STADE D: [4-(7-METHOXY-NAPHT-1-YL) BUTYRONITRILE

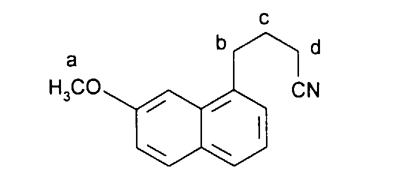

| **Réactifs :** | |
|---|---|
| Mésylate du 3-(7-methoxy-napht-1-yl) propan-1-ol | 3 g (10,2 x 10⁻³ mole) |
| KCN | 2 g (30,6 x 10⁻³ mole) |
| DMSO | 20 cm³ |

### Mode opératoire:

Dans une fiole de 100 cm³, dissoudre le mésylate du 3-(7-methoxy-napht-1-yl) propan-1-ol dans le DMSO. Ajouter KCN et mettre à reflux pendant 2h. Laisser refroidir. Verser dans un mélange eau / glace. Extraire à l'éther. Laver la phase organique avec de l'eau, la sécher sur CaCl₂ et l'évaporer. Recristalliser le produit obtenu.

| **Caractéristiques :** | |
|---|---|
| Masse molaire | 225,29 g/mole pour C₁₅H₁₅NO |
| Température de fusion | 52-54°C |
| Rf | 0,39 |
| Eluant | ATC (2/3/5) |
| Rendement | 90 % |
| Solvant de recristallisation | alcool /eau (1/5) |

| **Analyse spectroscopique dans l'infrarouge :** | |
|---|---|
| 3040-2820 cm⁻¹ | νCH alkyles |
| 2240 cm⁻¹ | vCN |
| 1620 et 1590 cm⁻¹ | vC = C |
| 1250 cm⁻¹ | νOCH₃ |

| **Analyse spectroscopique de RMN (300 MH**_{**z**}**, CDCl**_{**3**}**, δ)** | | | |
|---|---|---|---|
| 2,09 ppm (multiplet, 2H) | H_{c} | | |
| 2,35 ppm (triplet, 2H) | H_{d} | J_{d-c} | = 6,87 Hz |
| 3,18 ppm (triplet, 2H) | H_{b} | J_{b-c} | = 7,44 Hz |
| 3,94 ppm (singulet, 3H) | Hₐ | | |
| 7,16 ppm (doublet dédoublé, 1H) | H₆ | Jₒᵣₜₕₒ | = 8,91 Hz |
| | | J_{méta} | = 2,44 Hz |
| 7,23-7,30 ppm (massif, 3H) | H_{2,3,8} | | |
| 7,67 ppm (doublet dédoublé, 1H) | H₄ | Jₒᵣₜₕₒ | = 7,31 Hz |
| | | J_{méta} | = 1,77 Hz |
| 7,76 ppm (doublet, 1H) | H₅ | Jₒᵣₜₕₒ | = 8,91 Hz |

### STADE E : ACIDE 4-(7-METHOXY-NAPHT-1-YL)BUTYRIQUE

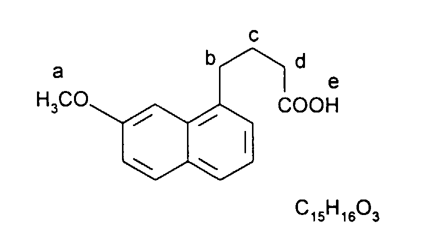

| **Réactifs :** | |
|---|---|
| 4-(7-méthoxy-napht-1-yl)butyronitrile | 1,8 g (8 x 10⁻³ mole) |
| NaOH 10 % | 3,5 cm³ (8 x 10⁻² mole) |
| Méthanol | 35 cm³ |
| HCI 1N | |

### Mode opératoire:

Dans un fiole de 100 cm³, dissoudre le nitrile dans le méthanol. Ajouter la soude. Mettre à reflux pendant 16 h. Laisser refroidir et acidifier grâce à HCI 1N. Filtrer et recristalliser le produit obtenu.

| **Caractéristiques :** | |
|---|---|
| Masse molaire | 244,29 g/mole pour C₁₅H₁₆O₃ |
| Température de fusion | 104-106°C |
| Rf | 0,54 |
| Eluant | ATC (4/4/2) |
| Rendement | 94 % |
| Solvant de recristallisation | toluène / cyclohexane (1/4) |

| **Analyse spectroscopique dans l'infrarouge :** | |
|---|---|
| 3000-2860 cm⁻¹ | νCH alkyle |
| 1700 cm⁻¹ | νCO acide |
| 1620 et 1590 cm⁻¹ | νC = C |
| 1240 cm⁻¹ | νOCH₃ |

| **Analyse spectroscopique de RMN (300 MH**_{**z**}**, CDCl**_{**3**}**, δ) :** | | | |
|---|---|---|---|
| 2,11 ppm (multiplet, 2H) | H_{c} | | |
| 2,51 ppm (triplet, 2H) | H_{d} | J_{d-c} | = 6,98 Hz |
| 3,09 ppm (triplet, 2H) | H_{b} | J_{b-c} | = 7,84 Hz |
| 3,95 ppm (singulet, 3H) | Hₐ | | |
| 7,15 ppm (doublet dédoublé, 1H) | H₆ | Jₒᵣₜₕₒ | = 8,94 Hz |
| | | J_{méta} | = 2,36 Hz |
| 7,23-7,39 ppm (massif, 3H) | H_{2,3,8} | | |
| 7,66 ppm (doublet dédoublé, 1H) | H₄ | Jₒᵣₜₕₒ | = 7,44 Hz |
| | | J_{méta} | = 1,65 Hz |
| 7,76 ppm (doublet, 1H) | H₅ | Jₒᵣₜₕₒ | = 8,94 Hz |
| H acide invisible dans CDCl₃ | | | |

### PREPARATION 3 : ACIDE 4-(5-FLUORO-INDOL-3-YL)BUTYRIQUE (J. Med. Chem. 1992, 35(22), pp 4020-4026)

### PREPARATION 4 : ACIDE 3-(5-METHYL-BENZOTHIOPHEN-3-YL)PROPANOIQUE (Monatsh. Chem. 1968, 99(2), pp 715-720)

### PREPARATION 5: ACIDE 3-(5-BENZYLOXY-BENZOTHIOPHEN-3-YL)PROPANOIQUE (J. Pharm. Pharmacol. 1973, 25(10), pp 847-848)

### PREPARATION 6: ACIDE 3-(5-PROPYL-INDOL-3-YL)PROPANOIQUE

### PREPARATION 7: ACIDE 3-(5-TRIFLUOROMETHYL-INDOL-3-YL)PROPANOIQUE (J. Amer. Chem. Soc. 1970, 92(10), pp en)

### PREPARATION 8 : ACIDE 3-(6-METHOXY-BENZOFUR-3-YL)PROPANOIQUE (C.R. Acad-Sci. Ser. C, 1970, 270(11), pp 1027-1029)

### PREPARATION 9 : ACIDE 4-(5-METHOXY-BENZOFUR-3-YL)BUTYRIQUE (Bull. Chem. Soc. Jpn 1976, 49(3), pp 737-740)

### PREPARATION 10 : ACIDE 4-(6-METHOXY-INDOL-3-YL)BUTYRIQUE (J. Chem. Soc., Chem. Commun., 1972, (8), pp 461-462)

### PREPARATION 11 : ACIDE 3-(5-ETHOXY-INDOL-3-YL)PROPANOIQUE (J. Karnatak Univ. 1972, 17, pp 33-42)

### PREPARATION 12 : ACIDE 4-(4,6-DIMETHOXY-INDOL-3-YL)BUTYRIQUE (J. Chem. Soc., Perkin Trans. 2, 1978, (8), pp 733-742)

### PREPARATION 13 : ACIDE 3-(5-HYDROXY-BENZOFUR-3-YL)PROPANOIQUE (Bull. Chem. Soc. Jpn 1982, 55(3), pp 865-869)

### EXEMPLE 1 : N-METHYL-2-(7-METHOXY-NAPHT-1-YL)ACETAMIDE

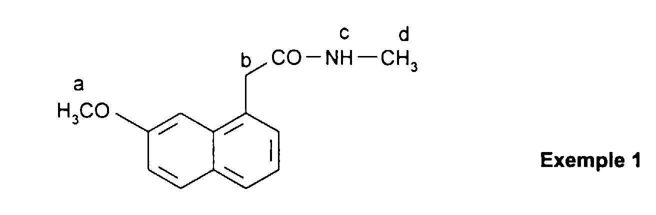

| **Réactifs :** | |
|---|---|
| Acide (7-méthoxy-napht-1-yl)acétique | 2 g (9,3 x 10⁻³ mole) |
| SOCl₂ | 6,75 cm³ (93 x 10⁻³ mole) |
| NH₂CH₃ | 3,18 cm³ (37,2 x 10⁻³ mole) |

### Mode opératoire :

Porter à reflux l'acide et le chlorure de thionyle pendant 1 h. Evaporer l'excès de SOCl₂. Reprendre par du CHCl₃ anhydre. Mettre le milieu dans un bain de glace et ajouter goutte à goutte l'amine. Laisser sous agitation pendant 2 h. Extraire la phase organique par de l'eau, du K₂CO₃ 10 %, de l'eau et de l'acide chlorhydrique 6N puis de l'eau. Sécher sur CaCl₂, évaporer. Recristalliser le résidu.

| **Caractéristiques :** | |
|---|---|
| Masse molaire | 229,28 g/mole pour C₁₄H₁₅NO₂ |
| Aspect | solide blanc |
| Température de fusion | 192-193°C |
| Rf | 0,48 |
| Eluant | ATC (4/4/2) |
| Rendement | 33 % |
| Solvant de recristallisation | toluène |

| **Analyse spectroscopique dans l'infrarouge :** | |
|---|---|
| 3280-3060 cm⁻¹ | νNH amide |
| 2920-2820 cm⁻¹ | νCH |
| 1640 cm⁻¹ | νCO amide |
| 1600 et 1590 cm⁻¹ | νC = C |
| 1260 cm⁻¹ | νOCH₃ |

| **Analyse spectroscopique de RMN (300 MH**_{**z**}**, CDCI**_{**3**}**, δ) :** | |
|---|---|
| 2,74 ppm (doublet, 3H) | H_{d} |
| 4,03 ppm (singulet, 3H) | Hₐ |
| 4,40 ppm (singulet, 2H) | H_{b} |
| 5,30 ppm (singulet, 1H) | H_{c} |
| 7,27-7,40 ppm (massif, 4H) | H_{2,3,6,8} |
| 7,78-7,84 ppm (massif, 2H) | H_{4,5} |

### EXEMPLE 2: N-METHYL-3-(7-METHOXY-NAPHT-1-YL)PROPIONAMIDE

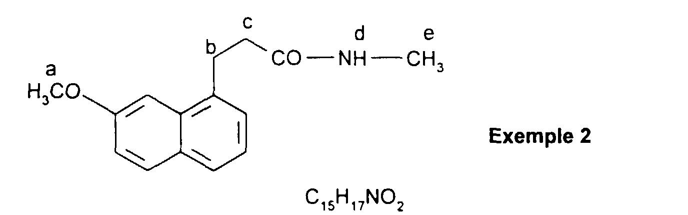

| **Réactifs :** | |
|---|---|
| Acide 3-(7-methoxy-napht-1-yl)-propanoïque | 0,3 g (1,3 x 10-3 mole) |
| SOCl₂ (118,97,d = 1,631) | 2,86 cm³ (2 x 1,95 x 10⁻² mole) |
| | |
| Méthylamine aqueuse 40 % (31,06,d = 0, 902) | 0,34 cm³ (3,9 x 10⁻³ mole) |

### Mode opératoire:

Dans une fiole de 100cm³, mettre 0,3 g de l'acide 3-(7-méthoxy-napht-1-yl) propanoïque et ajouter sous agitation SOCl₂. Laisser agiter 1 h. Evaporer l'excès de SOCl₂, ajouter du cyclohexane et laisser sous agitation 30 min. Filtrer et évaporer le filtrat. Ajouter la méthylamine d'un seul trait. Laisser sous agitation pendant 1 h. Extraire la phase aqueuse par 3 x 20 cm³ de toluène. Laver la phase organique par 3 x 10cm³ de K₂CO₃ 10% puis par 3 x 10 cm³ d'HCl 1N et laver à l'eau. Sécher sur CaCl₂ et évaporer à pression réduite. Recristalliser le solide obtenu.

| **Caractéristiques :** | |
|---|---|
| Masse molaire | 243, 31 g/mole pour C₁₅H₁₇NO₂ |
| Température de fusion | 98-100°C |
| Rf | 0,27 |
| Eluant | ATC (4/4/2) |
| Rendement | 78 % |
| Solvant de recristallisation | cyclohexane |

| **Analyse spectroscopique dans l'infrarouge :** | |
|---|---|
| 3060-3280 cm⁻¹ | νNH amide |
| 2880-2980 cm⁻¹ | νCH alkyles |
| 1630 cm⁻¹ | νC = O amide |
| 1590 cm⁻¹ | νC = C aromatique |
| 1250 cm⁻¹ | νOCH₃ |

| **Analyse spectroscopique de RMN (300 MH**_{**z**}**, CDCl**_{**3**}**, δ) :** | | | |
|---|---|---|---|
| 2,60 ppm (triplet, 2H) | H_{c} | J_{b-c} | = 7,68 H_{z} |
| 2,75 ppm (doublet, 3H) | Hₑ | J_{c-d} | = 4,77 H_{z} |
| 3,40 ppm (triplet, 2H) | H_{b} | J_{b-c} | = 7,68 H_{z} |
| 3,95 ppm (singulet, 3H) | Hₐ | | |
| 5,30 ppm (singulet, 1H) | pic large, NH | | |
| 7,13-7,35 ppm (massif, 4H) | H_{2,3,6,8} | | |
| 7,62 ppm (doublet, 1H) | H₄ | J ortho | = 7,49 H_{z} |
| 7,79 ppm (doublet, 2H) | H₅ | J orho | = 8,89 H_{z} |

### EXEMPLE 3 : N-METHYL 4-(7-METHOXY-NAPHT-1-YL)BUTYRAMIDE

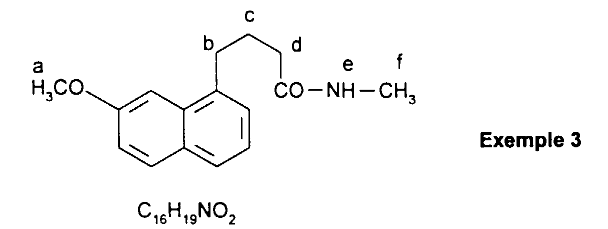

| **Réactifs :** | |
|---|---|
| Acide 4-(7-méthoxy-napht-1-yl)-butyrique | 1 g (4,1 x 10⁻³ mole) |
| NH₂CH₃ | 6,36 (82 x 10⁻³ mole) |
| SOCl₂ (118,97 d = 1,631) | 3 cm³ (41 x 10⁻³ mole) |
| cyclohexane anhydre | 30 cm³ |

### Mode opératoire :

Dans un ballon de 50 cm³, mettre l'acide, ajouter SOCl₂ et laisser sous agitation pendant 45 minutes. Ajouter 20 cm³ de cyclohexane et laisser agiter pendant 30 minutes puis filtrer et évaporer à sec le milieu. Reprendre par 10 cm³ de cyclohexane et ajouter NH₂CH₃ d'un seul tenant. Laisser sous agitation jusqu'à refroidissement du milieu puis filtrer le précipité blanc formé. Le résidu est recristallisé.

| **Caractéristiques :** | |
|---|---|
| Masse molaire | 257,33 g/mole pour C₁₆H₁₅NO₂ |
| Température de fusion | 96-98°C |
| Rf | 0,33 |
| Eluant | ATC (4/4/2) |
| Rendement | 93 % |
| Solvant de recristallisation | cyclohexane (puis eau / alcool) |

| **Analyse spectroscopique dans l'infrarouge :** | |
|---|---|
| 3300 cm⁻¹ | νNH amide |
| 2980-2800 cm⁻¹ | νCH |
| 1620 cm⁻¹ | νCO amide |
| 1590 cm⁻¹ | νC = C |
| 1250 cm⁻¹ | νOCH₃ |

| **Analyse spectroscopique de RMN (300 MH**_{**z**}**, CDCl**_{**3**}**, δ) :** | | | |
|---|---|---|---|
| 2,05 ppm (multiplet, 2H) | H_{c} | | |
| 2,30 ppm (triplet, 2H) | H_{d} | J_{d-c} | = 7,58 Hz |
| 2,80 ppm (doublet, 3H) | H_{f} | J_{f-e} | = 3,89 Hz |
| 3,51 ppm (triplet, 2H) | H_{b} | J_{b-c} | = 7,68 Hz |
| 4,03 ppm (singulet, 3H) | Hₐ | | |
| 5,61 ppm(signal, 1H) | Hₑ échangeable dans D₂O | | |
| 7,23-7,35 ppm (massif, 1H) | H_{2,3,6,8} | | |
| 7,66 ppm (doublet, 1H) | H₄ | Jₒᵣₜₕₒ | = 7,91 Hz |
| 7,78 ppm (doublet, 1H) | H₅ | Jₒᵣₜₕₒ | = 9,00 Hz |

### EXEMPLE 4 : N-PROPYL-4-(7-METHOXY-NAPHT-1-YL)BUTYRAMIDE

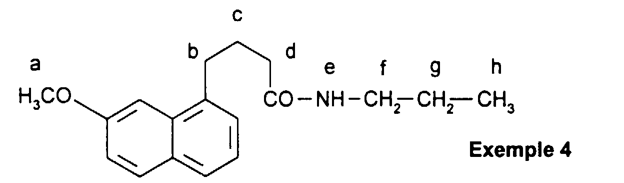

| **Réactifs :** | |
|---|---|
| Acide 4-(7-méthoxy-napht-1-yl)-butyrique | 1 g (4,1 x 10⁻³ mole) |
| SOCl₂ | 3 cm³ (41 x 10⁻³ mole) |
| NH₂C₃H₇ | 6,74 cm³ (8,2 x 10⁻³ mole) |

### Mode opératoire:

En procédant comme dans l'exemple 3 mais en remplaçant la méthylamine par le propylamine, on obtient le composé du titre.

| **Caractéristiques :** | |
|---|---|
| Masse molaire | 285,39 g/mole pour C₁₈H₂₃NO₂ |
| Température de fusion | 109-112°C |
| Rf | 0,51 |
| Eluant | ATC (4/4/2) |
| Rendement | 38 % |
| Solvant de recristallisation | eau / alcool (2/1) (puis cyclohexane) |

| **Analyse spectroscopique dans l'infrarouge :** | |
|---|---|
| 3300-3040 cm⁻¹ | νNH amide |
| 2940-2820 cm⁻¹ | νCH alkyle |
| 1630 cm⁻¹ | νCO amide |
| 1610 et 1590 cm⁻¹ | νC = C aromatique |
| 1260 cm⁻¹ | νOCH₃ |

| **Analyse spectroscopique de RMN (300 MHz, CDCl**_{**3**}**, δ) :** | | | |
|---|---|---|---|
| 0,91 ppm (triplet, 3H) | Hₕ | J_{h-g} | = 7,41 Hz |
| 1,50 ppm (multiplet, 2H) | H_{g} | | |
| 2,05 ppm (multiplet, 2H) | H_{c} | | |
| 2,27 ppm (triplet, 2H) | H_{d} | J_{d-c} | = 7,63 Hz |
| 3,20 ppm (triplet, 2H) | H_{f} | J_{f-g} | = 7,19 Hz |
| 3,50 ppm (triplet, 2H) | H_{b} | J_{b-c} | = 7,71 Hz |
| 4,02 ppm (singulet, 3H) | Hₐ | | |
| 5,61 ppm (signal, 1H) | Hₑ échangeable dans D₂O | | |
| 7,23-7,35 ppm (massif, 4H) | H_{2,3,6,8} | | |
| 7,67 ppm (doublet dédoublé, 1H) | H₄ | Jₒᵣₜₕₒ | = 7,99 Hz |
| | J_{méta} | | = 1,35 Hz |
| 7,77 ppm (doublet, 1H) | H₅ | Jₒᵣₜₕₒ | = 8,99 Hz |

### EXEMPLES 5 A 15 :

En procédant comme dans l'exemple 1, mais en utilisant l'amine appropriée, on obtient les composés du titre :
**EXEMPLE 5 : N-ETHYL-2-(7-METHOXY-NAPHT-1-YL)ACETAMIDE**
**EXEMPLE 6 : N-PROPYL-2-(7-METHOXY-NAPHT-1-YL)ACETAMIDE**
**EXEMPLE 7 : N-BUTYL-2-(7-METHOXY-NAPHT-1-YL)ACETAMIDE**
**EXEMPLE 8 : N-PENTYL-2-(7-METHOXY-NAPHT-1-YL)ACETAMIDE**
**EXEMPLE 9 : N-HEXYL-2-(7-METHOXY-NAPHT-1-YL)ACETAMIDE**
**EXEMPLE 10 : N-ISOPROPYL-2-(7-METHOXY-NAPHT-1-YL)ACETAMIDE**
**EXEMPLE 11 : N-ISOBUTYL-2-(7-METHOXY-NAPHT-1-YL)ACETAMIDE**
**EXEMPLE 12 : N(CYCLOPROPYLMETHYL)-2-(7-METHOXY-NAPHT-1-YL) ACETAMIDE**
**EXEMPLE 13 : N-(CYCLOBUTYL)-2-(7-METHOXY-NAPHT-1-YL)ACETAMIDE**
**EXEMPLE 14 : N-(CYCLOHEXYL)-2-(7-METHOXY-NAPHT-1-YL)ACETAMIDE**
**EXEMPLE 15 : N-ALLYL-2-(7-METHOXY-NAPHT-1-YL) ACETAMIDE**

### EXEMPLES 16 A 26 :

En procédant comme dans l'exemple 2, mais en utilisant l'amine appropriée, on obtient les composés du titre :
**EXEMPLE 16 : N-ETHYL-3-(7-METHOXY-NAPHT-1-YL)PROPIONAMIDE**
**EXEMPLE 17 : N-PROPYL-3-(7-METHOXY-NAPHT-1-YL)PROPIONAMIDE**
**EXEMPLE 18 : N-BUTYL-3-(7-METHOXY-NAPHT-1-YL)PROPIONAMIDE**
**EXEMPLE 19 : N-PENTYL-3-(7-METHOXY-NAPHT-1-YL)PROPIONAMIDE**
**EXEMPLE 20 : N-HEXYL-3-(7-METHOXY-NAPHT-1-YL)PROPIONAMIDE**
**EXEMPLE 21 : N-ISOPROPYL-3-(7-METHOXY-NAPHT-1-YL)PROPIONAMIDE**
**EXEMPLE 22 : N-ISOBUTYL-3-(7-METHOXY-NAPHT-1-YL)PROPIONAMIDE**
**EXEMPLE 23 : N-(CYCLOPROPYLMETHYL)-3-(7-METHOXY-NAPHT-1-YL) PROPIONAMIDE**
**EXEMPLE 24 : N-(CYCLOBUTYL)-3-(7-METHOXY-NAPHT-1-YL)PROPIONAMIDE**
**EXEMPLE 25 : N-(CYCLOHEXYL)-3-(7-METHOXY-NAPHT-1-YL)PROPIONAMIDE**
**EXEMPLE 26 : N-ALLYL-3-(7-METHOXY-NAPHT-1-YL)PROPIONAMIDE**

### EXEMPLES 27 A 36 :

En procédant comme dans l'exemple 3, mais en utilisant l'amine appropriée, on obtient les composés du titre :
**EXEMPLE 27 : N-ETHYL-4-(7-METHOXY-NAPHT-1-YL)BUTYRAMIDE Température de fusion : 123-128°C**
**EXEMPLE 28 : N-BUTYL-4-(7-METHOXY-NAPHT-1-YL)BUTYRAMIDE**
**EXEMPLE 29 : N-PENTYL-4-(7-METHOXY-NAPHT-1-YL)BUTYRAMIDE**
**EXEMPLE 30 : N-HEXYL-4-(7-METHOXY-NAPHT-1-YL)BUTYRAMIDE**
**EXEMPLE 31 : N-ISOPROPYL-4-(7-METHOXY-NAPHT-1-YL)BUTYRAMIDE**
**EXEMPLE 32 : N-ISOBUTYL-4-(7-METHOXY-NAPHT-1-YL)BUTYRAMIDE**
**EXEMPLE 33 : N-(CYCLOPROPYLMETHYL)-4-(7-METHOXY-NAPHT-1-YL) BUTYRAMIDE**
**EXEMPLE 34 : N-(CYCLOBUTYL)-4-(7-METHOXY-NAPHT-1-YL)BUTYRAMIDE**
**EXEMPLE 35 : N-(CYCLOHEXYL)-4-(7-METHOXY-NAPHT-1-YL)BUTYRAMIDE**
**EXEMPLE 36** : **N-ALLYL-4-(7-METHOXY-NAPHT-1-YL)BUTYRAMIDE**

### EXEMPLE 37 A 47 :

En procédant comme dans l'exemple 1, mais en utilisant les préparations 3 à 13, on obtient les composés des exemples suivants :
**EXEMPLE 37 : N-METHYL 4-(5-FLUORO-INDOL-3-YL)BUTYRAMIDE**
**EXEMPLE 38 : N-METHYL 3-(5-METHYL-BENZOTHIOPHEN-3-YL)PROPIONAMIDE**
**EXEMPLE 39 : N-METHYL 3-(5-BENZYLOXY-BENZOTHIOPHEN-3-YL) PROPIONAMIDE**
**EXEMPLE 40 : N-METHYL 3-(5-PROPYL-INDOL-3-YL)PROPIONAMIDE**
**EXEMPLE 41 : N-METHYL 3-(5-TRIFLUOROMETHYL-INDOL-3-YL)PROPIONAMIDE**
**EXEMPLE 42 : N-METHYL 3-(6-METHOXY-BENZOFUR-3-YL)PROPIONAMIDE**
**EXEMPLE 43 : N-METHYL 4-(5-METHOXY-BENZOFUR-3-YL)BUTYRAMIDE**
**EXEMPLE 44 : N-METHYL 4-(6-METHOXY-INDOL-3-YL)BUTYRAMIDE**
**EXEMPLE 45 : N-METHYL 3-(5-ETHOXY-INDOL-3-YL)PROPIONAMIDE**
**EXEMPLE 46 : N-METHYL 4-(4,6-DIMETHOXY-INDOL-3-YL)BUTYRAMIDE**
**EXEMPLE 47 : N-METHYL 3-(5-HYDROXY-BENZOFUR-3-YL)PROPIONAMIDE**

### EXEMPLE 48 A 58 :

En procédant comme dans les exemples précédents mais en utilisant les matières premières et les réactifs appropriés, on obtient les composés des exemples suivants :

### EXEMPLE 48 : N-METHYL 4-(7-HYDROXY NAPHT-1-YL)BUTYRAMIDE

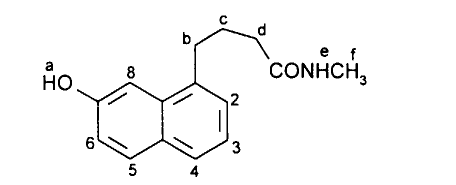

| **Réactifs :** | |
|---|---|
| N-méthyl-4-(7-méthoxy-napht-1-yl)-butyramide(exemple 3) | 1 g (3,9 x 10⁻³ mole) |
| Tribromure de bore | 1,12 cm³ (11,7 x 10⁻³ mole) |
| Dichlorométhane | 10 cm³ |

### Mode opératoire:

Dans une fiole de 100 cm³, dissoudre le N-méthyl-4-(7-méthoxy-napht-1-yl)butyramide dans le dichlorométhane. Placer la fiole dans un bain de glace à -5°C et laisser refroidir le milieu sous agitation pendant 20 minutes. Ajouter goutte à goutte le tribromure de bore. Au bout de 15 minutes, hydrolyser le milieu en le versant sur de l'eau glacée. Extraire par de l'acétate d'éthyle. Sécher cette phase sur MgSO₄ et l'évaporer. Purifier l'huile par chromatographie sur gel de silice.

| **Caractéristiques :** | |
|---|---|
| Masse molaire | 243,31 g /mole pour C₁₅H₁₇NO₂ |
| Aspect | huile |
| Rf | 0,40 |
| Eluant | ATC (4/4/2) |
| Rendement | 99 % |
| Purification l'eluant | chromatographie sur colonne avec cité ci-dessus |

| **Analyse spectroscopique dans l'infrarouge :** | |
|---|---|
| 3380-3080 cm⁻¹ | νNH amide et OH phénol |
| 2920-2840 cm⁻¹ | νCH alkyles |
| 1640 cm⁻¹ | νCO amide |
| 1610 et 1580 cm⁻¹ | νC = C aromatiques |

| **Analyse spectroscopique de RMN (80 MHz, CDCl**_{**3**}**, δ) :** | | | |
|---|---|---|---|
| 1,90-2,35 ppm (massif, 4H) | H_{c,d} | | |
| 2,70 ppm (doublet, 3H) | H_{f} | J_{f-e} | = 5,30 Hz |
| 2,90 ppm (triplet, 2H) | H_{b} | J_{b-c} | = 8,00 Hz |
| 5,85 ppm (signal, 1H) | Hₑ, échangeable dans D₂O | | |
| 7,00-7,80 ppm (massif, 6H) (Ha non visible) | H_{2,3,4,5,6,8} | | |

| **Analyse élémentaire :** | | | | |
|---|---|---|---|---|
| Calculé | C : 74,05% | H : 7,04% | O : 13,15% | N : 5,76% |
| Trouvé | C : 74,10% | H : 7,09% | O : 13,09% | N : 5,70% |

### EXEMPLE 49 : N-METHYL 4-(7-ALLYLOXY NAPHT-1-YL)BUTYRAMIDE

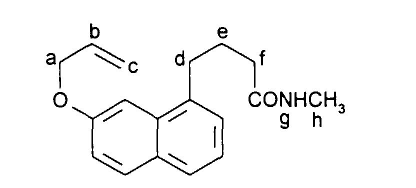

| **Réactifs :** | |
|---|---|
| N-méthyl-4-(7-hydroxy-napht-1-yl)-butyramide (exemple 48) | 2,15 g (8,8 x 10⁻³ mole) |
| Carbonate de potassium | 3,66 g (26,4 x 10⁻³ mole) |
| Bromure d'allyle | 3,82 cm³ (44,0 x 10⁻³ mole) |
| Acétone anhydre | 20 cm³ |

### Mode opératoire:

Dissoudre le dérivé naphtolique dans l'acétone anhydre. Ajouter le carbonate de potassium et laisser sous agitation à reflux pendant 30 minutes. Ajouter ensuite goutte à goutte le bromure d'allyle. Laisser à reflux et sous agitation pendant 3h. Après refroidissement, filtrer le milieu réactionnel et évaporer le filtrat. Recristalliser le résidu dans un solvant convenable.

| **Caractéristiques :** | |
|---|---|
| Masse molaire | 283,37 g /mole pour C₁₈H₂₁NO₂ |
| Aspect : | Solide blanc |
| Température de fusion | 119-122°C |
| Rf | 0,48 |
| Eluant | ATC (4/4/2) |
| Rendement | 60 % |
| Solvant de recristallisation | Toluène |

| **Analyse spectroscopique dans l'infrarouge :** | |
|---|---|
| 3240-3060 cm⁻¹ | νNH amide |
| 2920-2840 cm⁻¹ | νCH alkyles |
| 1630 cm⁻¹ | νCO amide |
| 1605 et 1580 cm⁻¹ | νC = C aromatiques |
| 1245 cm⁻¹ | νC -O aryl éther |

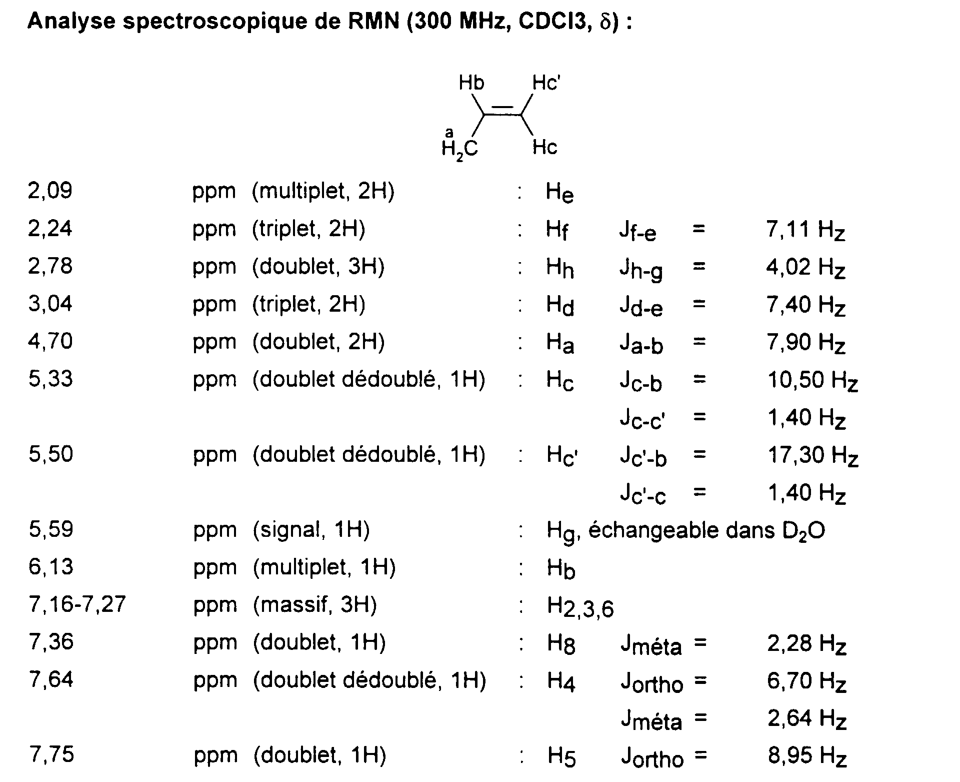

| **Analyse élémentaire :** | | | | |
|---|---|---|---|---|
| Calculé | C : 76,30% | H : 7,47% | O : 11,29% | N : 4,94% |
| Trouvé | C : 76,59% | H : 7,47% | O : 11,31% | N : 4,89% |

### EXEMPLE 50 : 1 N-METHYL 4-(8-ALLYL 7-HYDROXY NAPHT-1-YL)BUTYRAMIDE

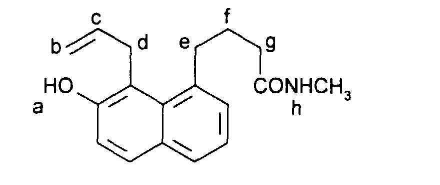

| **Réactifs :** | |
|---|---|
| N-méthyl-4-(7-allyloxy-napht-1-yl)-butyramide(exemple 49) | 1 g (3,5 x 10⁻³ mole) |
| N,N-diméthylaniline | 20 cm³ |

### Mode opératoire:

Dissoudre l'éther allylique dans la N,N-diméthylaniline et porter le milieu réactionnel à reflux (200°C) pendant 2h. Après refroidissement, ajouter 50 cm³ d'éther et extraire la phase organique par une solution aqueuse de soude à 10% puis par de l'eau. La phase aqueuse est ensuite acidifiée par une solution aqueuse de HCI 6N et laissée sous agitation pendant quelques minutes. Filtrer le précipité obtenu et le recristalliser dans un solvant convenable.

| **Caractéristiques :** | |
|---|---|
| Masse molaire | 283,37 g/mole pour C₁₈H₂₁NO₂ |
| Aspect | Solide blanc |
| Température de fusion : | 131-134°C |
| Rf | 0,38 |
| Eluant | ATC (4/4/2) |
| Rendement | 66 % |
| Solvant de recristallisation | Toluène |

| **Analyse spectroscopique dans l'infrarouge :** | |
|---|---|
| 3380 cm⁻¹ | νNH amide et OH phénol |
| 2940-2840 cm⁻¹ | νCH alkyles |
| 1630 cm⁻¹ | νCO amide |
| 1605 et 1590 cm⁻¹ | νC = C aromatiques |

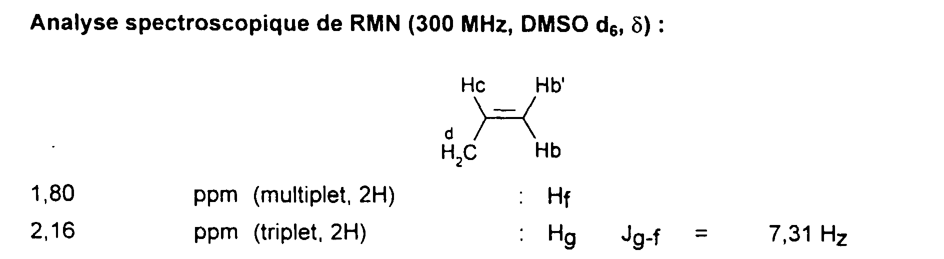

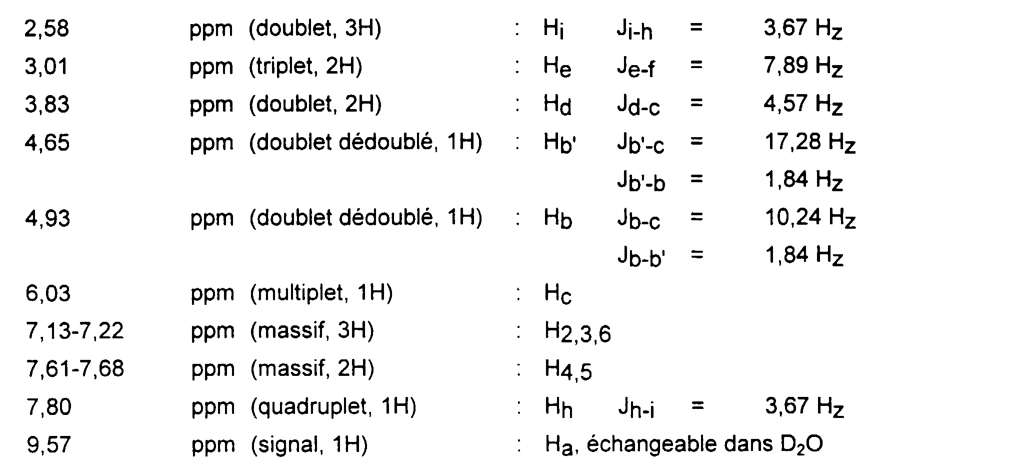

| **Analyse élémentaire :** | | | | |
|---|---|---|---|---|
| Calculé | C : 76,30% | H : 7,47% | O : 11,29% | N : 4,94% |
| Trouvé | C : 76,48% | H : 7,53% | O : 11,25% | N : 4,85% |

### EXEMPLE 51 : N-METHYL 4-(8-ALLYL 7-METHOXY NAPHT-1-YL)BUTYRAMIDE

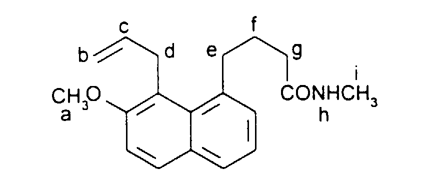

| **Réactifs :** | |
|---|---|
| N-méthyl-4-(8-allyl-7-hydroxy-napht-1-yl)-butyramide (exemple 50) | 1 g (3,5 x 10⁻³ mole) |
| Diméthylsulfate | 3,35 cm³ (35,0 x 10⁻³ mole) |
| Solution aqueuse de soude à 10% | 14 cm³ (35,0 x 10⁻³ mole) |

### Mode opératoire:

Dissoudre le dérivé N-méthyl-4-(8-allyl-7-hydroxy-napht-1-yl)-butyramide dans une solution aqueuse de soude à 10% à 50°C. Arrêter le chauffage au bout de 30 minutes et laisser refroidir, puis ajouter goutte à goutte le diméthylsulfate. Laisser sous agitation pendant 30 minutes et extraire la phase aqueuse basique par 3 fois 20 cm³ d'éther. Laver la phase organique par de l'eau jusqu'à neutralité des eaux de lavage. Sécher la phase organique sur du MgSO₄, la filtrer et l'évaporer au bain-marie sous vide. Recristalliser le résidu dans un solvant convenable.

| **Caractéristiques :** | |
|---|---|
| Masse molaire | 297,40 g/mole pour C₁₉H₂₃NO₂ |
| Aspect : | Solide blanc |
| Température de fusion | 84-86°C |
| Rf | 0,51 |
| Eluant | ATC (4/4/2) |
| Rendement | 68 % |
| Solvant de recristallisation | Toluène - Cyclohexane (1-2) |

| **Analyse spectroscopique dans l'infrarouge :** | |
|---|---|
| 3240 et 3060 cm⁻¹ | νNH amide |
| 2980-2810 cm⁻¹ | νCH alkyles |
| 1620 cm⁻¹ | νCO amide |
| 1600 et 1580 cm⁻¹ | νC = C aromatiques |
| 1240 cm⁻¹ | νOCH₃ aryl éther |

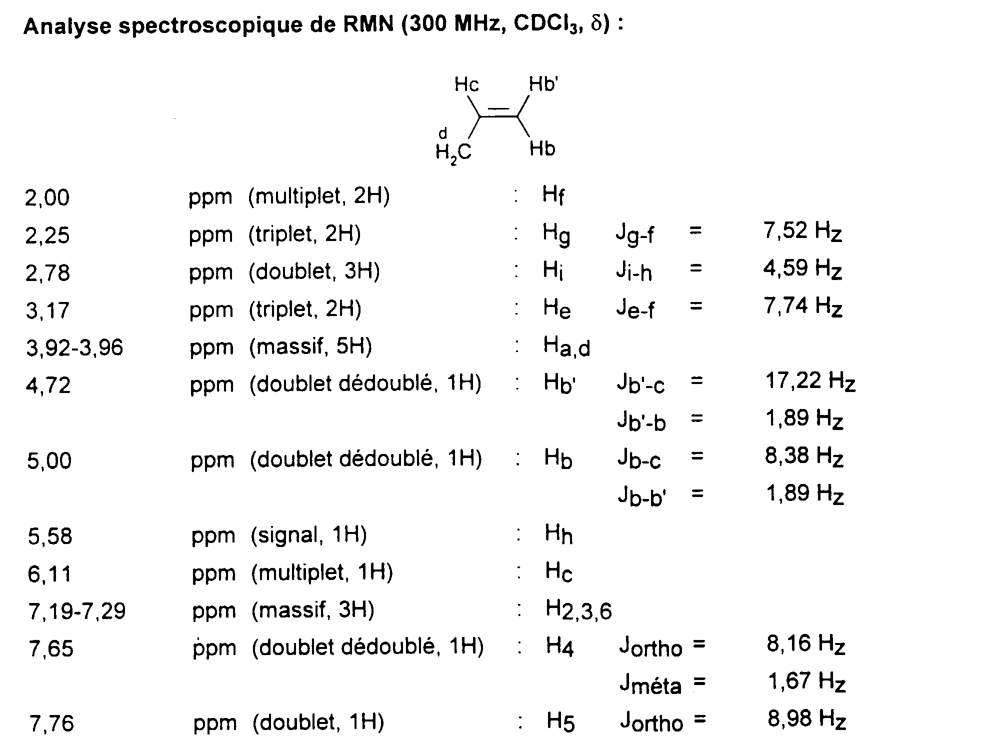

| **Analyse élémentaire :** | | | | |
|---|---|---|---|---|
| Calculé | C : 76,74% | H : 7,80% | O : 10,76% | N : 4,71% |
| Trouvé | C : 76,94% | H : 7,99% | O : 10,78% | N : 4,72% |

### EXEMPLE 52 : N-METHYL 4-(8-ALLYL 7-PROPOXY NAPHT-1-YL)BUTYRAMIDE

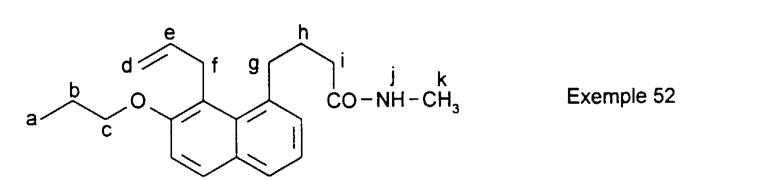

| **Réactifs :** | |
|---|---|
| N-méthyl-4-(8-allyl-7-hydroxy-napht-1-yl)-butyramide (exemple 50) | 0,94 g (3,3 x 10-3 mole) |
| Carbonate de potassium | 1,37 g (10,0 x 10⁻³ mole) |
| 1-Iodopropane | 1,63 cm³ (16,5 x 10⁻³mole) |
| Acétone anhydre | 20 cm³ |

### Mode opératoire :

Dissoudre le dérivé naphtolique dans l'acétone anhydre. Ajouter le carbonate de potassium et laisser sous agitation à reflux pendant 30 minutes. Ajouter ensuite goutte à goutte l'iodopropane. Laisser à reflux et sous agitation pendant 3h. Après refroidissement, filtrer le milieu réactionnel et évaporer le filtrat. Recristalliser le résidu dans un solvant convenable.

| **Caractéristiques :** | |
|---|---|
| Masse molaire | 325,45 g/mole pour C₂₁H₂₇NO₂ |
| Aspect | Solide blanc |
| Température de fusion | 69-71°C |
| Rf | 0,53 |
| Eluant | ATC (4/4/2) |
| Rendement | 82 % |
| Solvant de recristallisation | Cyclohexane |

| **Analyse spectroscopique dans l'infrarouge :** | |
|---|---|
| 3260 cm⁻¹ | νNH amide |
| 2940-2840 cm⁻¹ | νCH alkyles |
| 1625 cm⁻¹ | νCO amide |
| 1600 et 1590 cm⁻¹ | νC = C aromatiques |
| 1250 cm⁻¹ | νC-O aryl éther |

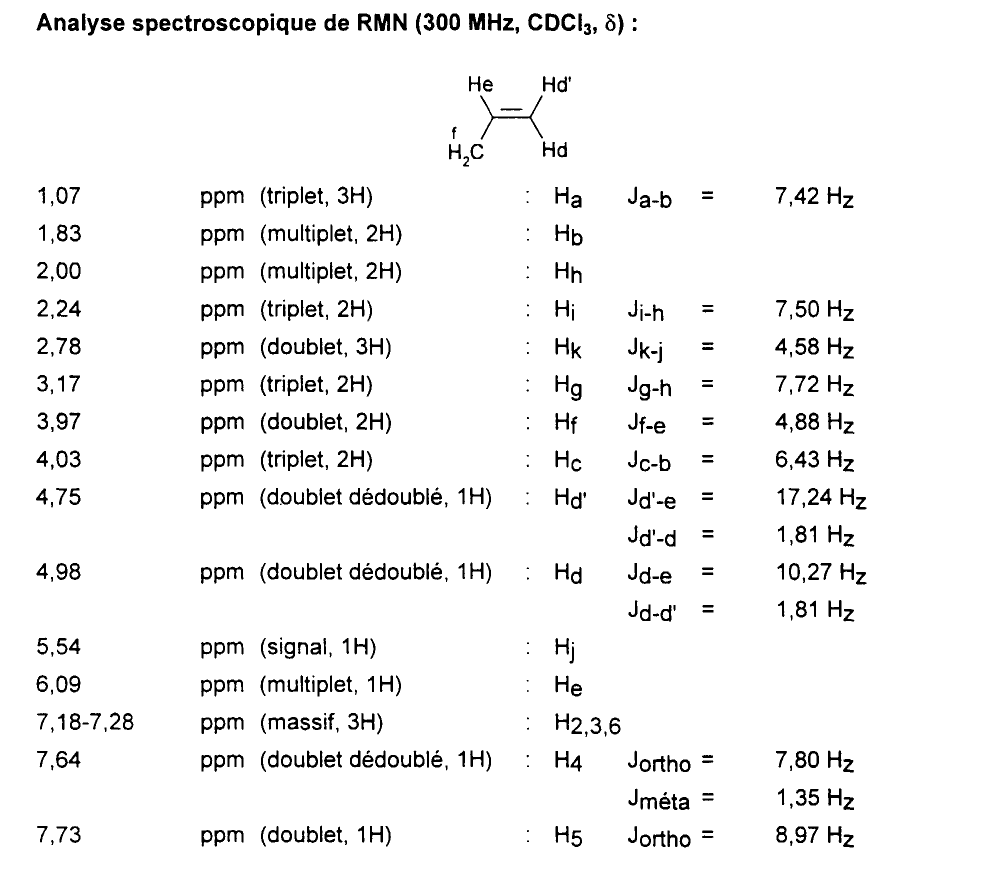

| **Analyse élémentaire :** | | | | |
|---|---|---|---|---|
| Calculé | C : 77,50% | H : 8,36% | O : 9,83% | N : 4,30% |
| Trouvé | C : 77,43% | H : 8,27% | O : 9,80% | N : 4,20% |

### EXEMPLE 53 : N-METHYL 4-(7-PROPOXY NAPHT-1-YL)BUTYRAMIDE Température de fusion : 102-105°C

### EXEMPLE 54 : N-METHYL 4-(2,3-DIHYDRO-3-OXO-1-NAPHTO[1, 2-b] FURANNE)BUTYRAMIDE

### EXEMPLE 55 : N-METHYL 4-(2,3-DIHYDRO-3-HYDROXY-1-NAPHTO[1,2-b] FURANNE)BUTYRAMIDE

### EXEMPLE 56 : N-METHYL 4-(1-NAPHTO[1,2-b]FURANNE)BUTYRAMIDE

### EXEMPLE 57 : N-METHYL 4-(2H-1-NAPHTO[1,2-b]PYRANNE)BUTYRAMIDE

### EXEMPLE 58 : N-PROPYL 4-(2,3-DIHYDRO-3-OXO-1-NAPHTO[1,2-b] FURANNE)BUTYRAMIDE

### EXEMPLE 59 : N-PROPYL 4-(2,3-DIHYDRO-3-HYDROXY-1-NAPHTO[1,2-b] FURANNE)BUTYRAMIDE

### EXEMPLE 60 : N-PROPYL 4-(1-NAPHTO[1,2-b]FURANNE)BUTYRAMIDE

### EXEMPLE 61 : N-PROPYL 4-(2H-1-NAPHTO[1,2-b]PYRANNE)BUTYRAMIDE

### ETUDE PHARMACOLOGIQUE

### EXEMPLE A : ETUDE DE LA TOXICITE AIGUE

La toxicité aigüe a été appréciée après administration orale à des lots de 8 souris (26 ± 2 grammes). Les animaux ont été observés à intervalles réguliers au cours de la première journée et quotidiennement pendant les deux semaines suivant le traitement. La DL₅₀, entraînant la mort de 50 % des animaux, a été évaluée.

La DL₅₀ des produits testés est supérieure à 1 000 mg.kg⁻¹ pour la plupart des composés étudiés, ce qui indique la faible toxicité des composés de l'invention.

### EXEMPLE B : ETUDE DE LIAISON AUX RECEPTEURS DE LA MELATONINE

### B1 ) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology 1989, vol. (1), pp 1-4).

### PROTOCOLE

1) Les membranes de pars tuberalis de mouton sont préparées et utilisées comme tissu cible dans des expériences de saturation pour déterminer les capacités et affinités de liaison pour la 2-[¹²⁵l]-iodomélatonine.
2) Les membranes de pars tuberalis de mouton sont utilisées comme tissu cible, avec les différents composés à tester, dans des expériences de liaison compétitive par rapport à la 2-iodomélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

Il apparaît que les composés de l'invention possèdent une puissante affinité pour les récepteurs de la mélatonine supérieure à celle de la mélatonine elle-même.

### B2) Etude sur des membranes de cellules du cerveau de poulet (Gallus domesticus)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à -200°C puis conservés à -80°C. Les membranes sont préparées selon la méthode décrite par Yuan et Pang (Journal of Endocrinology 1991, **128**, pp 475-482). La 2-[¹²⁵l]-iodomélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman ® LS 6000.
Les produits utilisés sont :
- 2-[¹²⁵l]-iodomélatonine
- mélatonine
- produits courants
- composés testés

En screening primaire, les molécules sont testées à 2 concentrations (10⁻⁷ et 10⁻⁵M). Chaque résultat est la moyenne de n=3 mesures indépendantes. Les composés testés font l'objet d'une détermination quantitative de leur efficacité (IC₅₀). Ils sont utilisés à 10 concentrations différentes.

Ainsi, les valeurs d'IC₅₀ trouvées pour les composés préférés de l'invention, qui correspondent aux valeurs de l'affinité, montrent que la liaison des composés testés est très puissante.

### EXEMPLE C : TEST DES QUATRE PLAQUES

Les produits de l'invention sont administrés par voie oesophagienne à des lots de dix souris. Un lot reçoit du sirop de gomme. 30 min après administration des produits à étudier, les animaux sont placés dans des habitacles dont le plancher comprend quatre plaques métalliques. Chaque fois que l'animal passe d'un plaque à l'autre, il reçoit une légère décharge électrique (0,35 mA). Le nombre de passage est enregistré pendant une minute. Après administration, les composés de l'invention augmentent de façon significative le nombre de passages ce qui montre l'activité anxiolytique des dérivés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir, le rat placé en isolement temporel (obscurité permanente).

### PROTOCOLE EXPERIMENTAL

Des rats mâles Long Evans âgés de un mois sont soumis dès leur arrivée au laboratoire, à un cycle lumineux de 12 h de lumière par 24 h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité grâce au rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre-cours et pendant le traitement,
- de mettre éventuellement en évidence, par analyse spectrale, l'existence de composants circadiens et non circadiens.

### RESULTATS:

II apparaît clairement que les composés de l'invention permettent d'agir de façon puissante sur le rythme circadien via le système mélatoninergique.

### EXEMPLE E: ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO₂/N₂ 5/95 %. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesure simultanément :
- sur le crémaster perfusé normalement,
- sur le crémaster sous ligature, c'est à dire le crémaster ischémié 2, 7, 14 et 21 jours après ligature.

Deux groupes d'animaux ont été étudiés :
- un groupe témoin sans traitement,
- un groupe traité per os par un produit de l'invention, à raison de 0,1 mg.kg⁻¹ par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

### EXEMPLE F : STIMULATION DES REPONSES IMMUNITAIRES

A des groupes de six souris, on a administré des globules rouges de mouton. Ces groupes de souris ont ensuite été traités par voie sous cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

### EXEMPLE G : INHIBITION DE L'OVULATION

On utilise des rates femelles adultes avec des cycles réguliers de quatre jours.

Des frottis vaginaux quotidiens ont été réalisés et des rates ont été sélectionnés après qu'elles ont montré au moins deux cycles consécutifs de quatre jours.

Chaque cycle est constitué de deux jours de dioestrus, un jour de proestrus et un jour d'oestrus.

L'après-midi du jour de proestrus, l'hormone lutéinisante est libérée dans le sang par l'hypophyse. Cette hormone induit l'ovulation qui se traduit par la présence d'oeufs au niveau de l'oviducte le jour de l'oestrus. Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées.

### EXEMPLE H : ACTIVITE ANTIARRYTHMIQUE

PROTOCOLE (ref: Lawson J.N. et al. J. Pharmacol. Exper. Therap. 1968, 160, pp 22-31) La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arrythmies et de fréquences cardiaques supérieures à 200 battements/min (témoin : 400480 battements/min) chez les animaux indique une protection significative.

### EXEMPLE I : ACTIVITE ANTI-AGREGANTE PLAQUETTAIRE

### PROTOCOLE

### (Ref. : Bertele V. et al. Science. 220 : 517-519, 1983 Ibid, Eur. J. Pharmacol. 85 : 331-333, 1982)

Les composés de l'invention (100 µg/ml) sont testés pour leur capacité à inhiber l'agrégation plaquettaire irréversible induite par l'arachidonate de sodium (50 µg/ml) dans du plasma de lapin enrichi en plaquettes.

Une inhibition de plus de 50 % de l'agrégation maximum indique une activité significative pour les composés de l'invention.

Ce test *in vitro* montre que les composés de l'invention sont de bons candidats pour le traitement des maladies cardiovasculaires, notamment les thromboses

### EXEMPLE J : PROLONGATION DU TEMPS DE SAIGNEMENT

### PROTOCOLE

### (Ref. : Djana E. et al. Thrombosis Research. 15 : 191-197, 1979) Butler K.D. et al. Thromb. Haemostasis. 47 : 4649, 1982)

Les composés à tester sont administrés par voie orale (100 mg/kg) à un groupe de 5 souris 1h avant le sectionnement standardisé du bout de chaque queue (0,5 mm).

Les souris sont immédiatement suspendues verticalement, les queues étant immergées de 2 cm dans un tube à essai contenant une solution saline isotonique à 37°C.

Le temps requis pour que le saignement cesse pendant une période de 15 secondes est alors déterminé.

Une prolongation de plus de 50 % du temps de saignement relative à un groupe d'animaux contrôle est considérée comme significative pour les composés de l'invention.

Ce test *in vivo* confirme l'intérêt des composés de l'invention pour le traitement des pathologies cardiovasculaires puisque les composés de l'invention prolongent le temps de saignement.

### EXEMPLE K: TEST D'HYPOXIE HYPOBARE

### PROTOCOLE

### (Ref. : Gotti B., et Depoortere H., Circ. Cerebrale, Congrès de Circulation Cérébrale, Toulouse, 105-107, 1979)

Les composés à tester sont administrés par voie intrapéritonéale (100 mg/kg) à un groupe de 3 souris 30 minutes avant d'être placés dans une chambre à la pression hypobare de 20 cm Hg.

La prolongation du temps de survie par rapport à un groupe d'animaux traités avec le véhicule de plus de 100 % en absence d'effet dépresseur du système nerveux central indique une activité cérébroprotective des composés de l'invention.

### EXEMPLE L: COMPOSITION PHARMACEUTIQUE : COMPRIMES

1000 comprimés dosés à 5 mg de N-méthyl4-(7-méthoxy-napht-1-yl)butyramide

| | |
|---|---|
| N-méthyl-4-(7-méthoxy-napht-1-yl)butyramide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I): 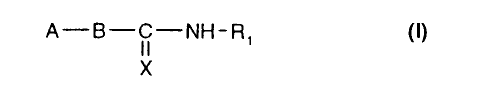 dans laquelle :
- A représente un groupement de formule : 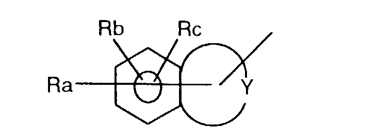
dans laquelle :
- Y forme avec le noyau benzo qui le porte un cycle choisi parmi naphtalène, dihyd ronaphtalène, tétrahyd ronaphtalène, benzofurane, 2,3-dihydrobenzofurane, benzothiophène, 2,3-dihydrobenzothiophène, indole, indoline, 2H-indène et indane ;
- Ra, Rb et Rc, chacun indépendamment l'un de l'autre, représentent un hydrogène ou un radical choisi parmi halogène, hydroxy, -Rd et -O-Rd ; avec Rd étant choisi parmi alkyle, alkyle substitué par un ou plusieurs halogènes, alcényle, alcynyle, cycloalkyle, cycloalkylalkyle, aryle, arylalkyle, aryle substitué, arylalkyle substitué ;
Ra pouvant également former avec Rb et les 2 atomes de carbone adjacents qui les portent, un cycle A₁ choisi parmi furane, dihydrofurane, pyrane et dihydropyrane, A₁ étant éventuellement substitué par un ou plusieurs radicaux ou groupements choisis parmi hydroxy, alkyle, alkoxy et oxo ;
- B représente une chaîne alkylène de 2 à 6 atomes de carbone non substituée ou substituée par un ou plusieurs radicaux choisis parmi alkyle, alkoxy, hydroxy, carboxy, alkoxycarbonyle, carboxyalkyle et alkoxycarbonylalkyle étant entendu que B peut également représenter une chaîne méthylène lorsque Y forme avec le noyau benzo qui le porte un naphtalène, un dihydronaphtalène ou un tétrahydronaphtalène et que Ra, Rb et Rc ne représentent pas simultanément des hydrogènes ;
avec la réserve que Ra, Rb et Rc ne peuvent représenter simultanément des hydrogènes ou un hydrogène et 2 radicaux méthyle ou 2 hydrogènes et un radical méthyle, lorsque Y forme avec le noyau benzo auquel il est lié un indole,
et que le composé de formule (I) ne peut pas être le N-méthyl-3-(4-méthoxy-napht-1-yl)propionamide ni le N-tertbutyl-3-carboxy-4-(napht-1-yl)butyramide,
- X représente un oxygène ou un soufre ;
- et R₁ représente un radical choisi parmi alkyle, alkyle substitué par un ou plusieurs halogènes, alcényle, alcynyle, cycloalkyle et cycloalkylalkyle,
étant entendu que :
lorsque Y forme avec le noyau benzo qui le porte un cycle naphtalène, B représente une chaîne alkylène de 2 à 6 atomes de carbone, X représente un atome d'oxygène, 2 des groupements Ra, Rb ou Rc représentent simultanément un atome d'hydrogène et le troisième représente un groupement hydroxy ou alkoxy (C₁-C₆) linéaire ou ramifié (Ra est dans ce cas un substituant de la partie benzo),
alors R₁ ne peut représenter un groupement alkyle ou alkyle substitué par un groupement cycloalkyle,
étant entendu que :
- les termes "alkyle" et "alkoxy" désignent des groupements linéaires ou ramifiés contenant de 1 à 6 atomes de carbone,
- les termes "alcényle" et "alcynyle" désignent des groupements insaturés linéaires ou ramifiés contenant de 2 à 6 atomes de carbone,
- le terme "cycloalkyle" désigne un groupement de 3 à 8 atomes de carbone,
- le terme "aryle" désigne un radical phényle, naphtyle ou pyridyle,
- le terme "substitué" affecté aux expressions "aryle" et "arylalkyle" signifie que ces groupements peuvent être substitués par un ou plusieurs radicaux choisis parmi halogène, alkyle, alkoxy, hydroxy et alkyle substitué par un ou plusieurs halogènes ;
leurs énantiomères et diastéréoisomères,
et leurs sels d'addition à un acide ou une base pharmaceutiquement acceptable.

2. Composé de formule (I) selon la revendication 1 qui est le N-méthyl-2-(7-méthoxynapth-1-yl)acétamide.

3. Composé de formule (I) selon la revendication 1 qui est le N-méthyl-3-(7-méthoxynapht-1-yl)propionamide.

4. Composé de formule (I) selon la revendication 1 qui est le N-propyl-4-(7-méthoxynapht-1-yl)butyramide.

5. Procédé de préparation des composés de formule (I) selon la revendication 1 caractérisé en ce que on fait réagir un acide de formule (II):
A―B―COOH **(II)**
dans laquelle A et B sont tels que définis dans la revendication 1,
avec une amine de formule (III):
H₂N―R₁ **(III)**
dans laquelle R₁ est tel que défini dans la revendication 1,
pour obtenir le composé de formule (I/a): 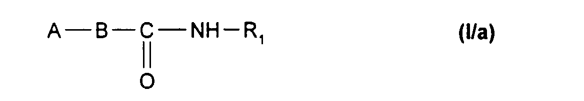 dans laquelle A, B et R₁ sont tels que définis précédemment,
composé de formule (I/a) qui, traité par un réactif de thionation, donne le composé de formule (I/b) : 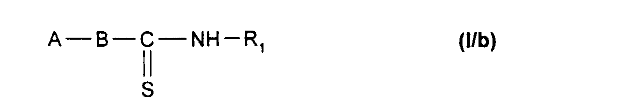 dans laquelle A, B et R₁ sont tels que définis précédemment,
les composés de formule (I/a) et (I/b) formant l'ensemble des composés de formule (I) selon la revendication 1 qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

6. Procédé de préparation des composés de formule (I/c) cas particulier des composés de formule (I) selon la revendication 1 : 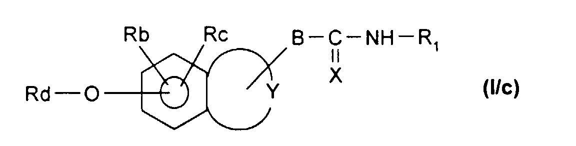 dans laquelle B, X, Y, R₁, R_{b}, R_{c} et R_{d} sont tels que définis dans la revendication 1 caractérisé en ce que l'on greffe le radical R_{d} sur un composé de formule (l/d): 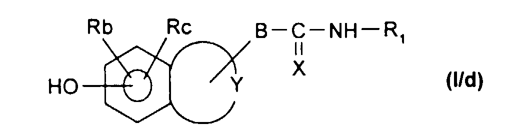 dans laquelle B, X, Y, R₁, R_{b} et R_{c} sont tels que définis précédemment.
composés de formule (I/c) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

7. Procédé de préparation des composés de formule (l/j): 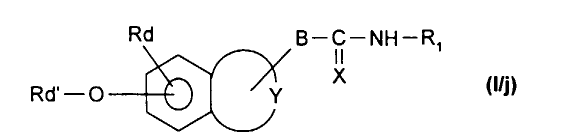 dans laquelle Rd, Y, X, B, et R₁ sont tels que définis dans la revendication 1 et Rd' peut prendre les même valeurs que Rd tel que défini précédemment,
caractérisé en ce que on greffe le radical Rd tel que défini précédemment sur la fonction hydroxy d'un composé de formule (l/k): 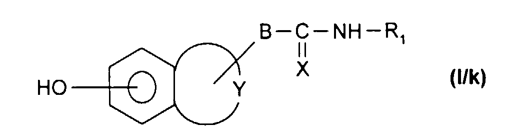 dans laquelle Y, B, X, et R₁ sont tels que définis précédemment pour obtenir un composé de formule (I/ℓ) : 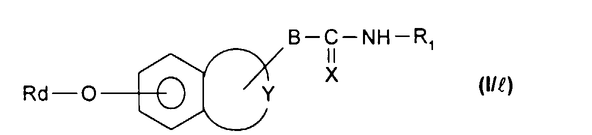 dans laquelle Rd, Y, B, X et R₁ sont tels que définis précédemment,
composé de formule (l/ℓ) qui est ensuite soumis à une réaction de transposition pour obtenir le composé de formule (l/m): 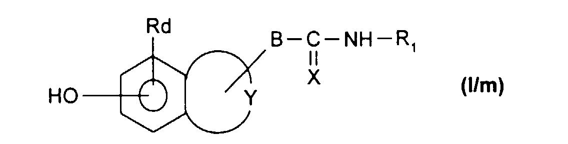 dans laquelle Rd, Y, B, X et R₁ sont tels que définis précédemment,
la fonction hydroxy étant de nouveau greffée par un radical Rd' tel que défini précédemment pour obtenir un composé de formule (l/j): 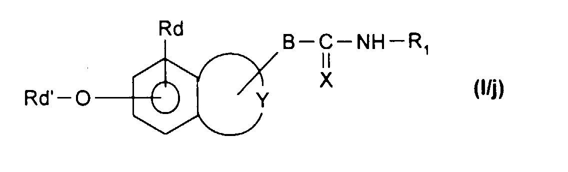 dans laquelle Rd, Rd', Y, B, X et R₁ sont tels que définis précédemment,
les composés de formule (I/j) qui peuvent être, si on le désire,
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- et/ou salifiés par un acide ou une base pharmaceutiquement acceptable.

8. Compositions pharmaceutiques contenant un composé de formule (I) selon la revendication 1 en combinaison avec un ou plusieurs excipients pharmaceutiquement acceptables.

9. Compositions pharmaceutiques selon la revendication 8 utiles pour le traitement des troubles du système mélatoninergique et des troubles liés au système mélatoninergique.

10. Compositions selon la revendication 8 ou 9 utiles dans le traitement des dépressions saisonnières, des troubles du sommeil, des pathologies cardiovasculaires, des insomnies et fatigues dues aux décalages horaires, des troubles de l'appétit et de l'obésité.

11. Utilisation pour l'obtention de compositions pharmaceutiques destinées au traitement des troubles du système mélatoninergique et des troubles liés au système mélatoninergique, des composés de formule (I_{A}): 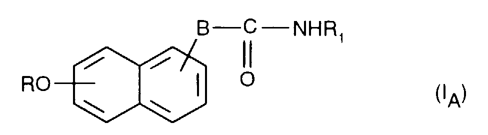 dans laquelle :
- R représente un atome d'hydrogène ou un groupement alkyle (C₁-C₆) linéaire ou ramifié,
- B représente une chaîne alkylène de 2 à 6 atomes de carbone,
- R₁ représente un groupement alkyle (C₁-C₆) linéaire ou ramifié non substitué ou substitué par un ou plusieurs groupements cycloalkyle (C₃-C₈),
leurs énantiomères et diastéréoisomères,
ainsi que leurs sels d'addition à un acide ou à une base pharmaceutiquement acceptable.

12. Utilisation pour l'obtention de compositions pharmaceutiques destinées au traitement des dépressions saisonnières, des troubles du sommeil, des pathologies cardiovasculaires, des insomnies et fatigues dues aux décalages horaires, des troubles de l'appétit et de l'obésité, des composés de formule (I_{A}): 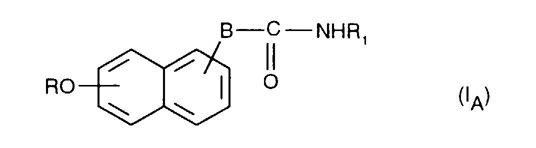 dans laquelle :
- R représente un atome d'hydrogène ou un groupement alkyle (C₁-C₆) linéaire ou ramifié,
- B représente une chaîne alkylène de 2 à 6 atomes de carbone,
- R₁ représente un groupement alkyle (C₁-C₆) linéaire ou ramifié non substitué ou substitué par un ou plusieurs groupements cycloalkyle (C₃-C₈),
leurs énantiomères et diastéréoisomères,
ainsi que leurs sels d'addition à un acide ou à une base pharmaceutiquement acceptable.

## Patentansprüche

1. Verbindungen der Formel (I): 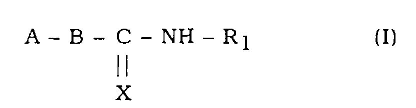 in der:
- A eine Gruppe der Formel: 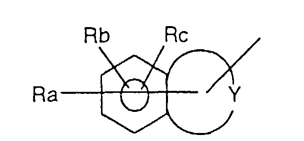 in der:
- Y mit dem sie tragenden Benzokern einen Ring ausgewählt aus Naphthalin, Dihydronaphthalin, Tetrahydronaphthalin, Benzofuran, 2,3-Dihydrobenzofuran, Benzothiophen, 2,3-Dihydrobenzothiophen, Indol, Indolin, 2H-Inden und Indan bildet;
- Ra, Rb und Rc jeweils unabhängig voneinander Wasserstoff oder eine Gruppe ausgewählt aus Halogen, Hydroxy, -Rd und -O-Rd darstellen; worin Rd ausgewählt ist aus Alkyl, Alkyl substituiert durch ein oder mehrere Halogene, Alkenyl, Alkinyl, Cycloalkyl, Cycloalkylalkyl, Aryl, Arylalkyl, substituiertem Aryl und substituiertem Arylalkyl;
worin Ra zusammen mit Rb und den 2 sie tragenden benachbarten Kohlenstoffatomen einen Ring A₁ bilden kann ausgewählt aus Furan, Dihydrofuran, Pyran und Dihydropyran, worin A₁ gegebenenfalls durch ein oder mehrere Reste oder Gruppen ausgewählt aus Hydroxy, Alkyl, Alkoxy und Oxo substituiert ist;
- B eine Alkylenkette mit 2 bis 6 Kohlenstoffatomen, die nicht substituiert oder durch eine oder mehrere Gruppen, ausgewählt aus Alkyl, Alkoxy, Hydroxy, Carboxy, Alkoxycarbonyl, Carboxyalkyl und Alkoxycarbonylalkyl, bedeutet, wobei B auch eine Methylenkette darstellen kann, wenn Y mit dem sie tragenden Benzokern eine Naphthalin-, Dihydronaphthalin- oder Tetrahydronaphthalingruppe bildet, und daß Ra, Rb und Rc nicht gleichzeitig Wasserstoff bedeuten;
mit der Maßgabe, daß Ra, Rb und Rc nicht gleichzeitig Wasserstoff bedeuten oder ein Wasserstoff und 2 Methylgruppen oder 2 Wasserstoffe und eine Methylgruppe darstellen, wenn Y mit dem sie tragenden Benzokern eine Indolgruppe bildet, und daß die Verbindung der Formel (I) nicht N-Methyl-3-(4-methoxy-naphth-1-yl)-propionamid noch N-tert.-Butyl-3-carboxy-4-(naphth-1-yl)-butyramid-ist,
- X Sauerstoff oder Schwefel darstellt;
- und R₁ eine Gruppe ausgewählt aus Alkyl, Alkyl substituiert durch ein oder mehrere Halogene, Alkenyl, Alkinyl, Cycloalkyl und Cycloalkylalkyl darstellt,
mit der Maßgabe, daß:
wenn Y mit dem sie tragenden Benzokern einen Naphthalinring bildet, B eine Alkylenkette mit 2 bis 6 Kohlenstoffatomen darstellt, X ein Sauerstoffatom darstellt, 2 der Gruppen Ra, Rb oder Rc gleichzeitig ein Wasserstoffatom und die dritte eine Hydroxygruppe oder eine geradkettige oder verzweigte (C₁-C₆)-Alkoxygruppe bedeutet (worin Ra in diesem Fall ein Substituent des Benzorests ist),
dann R₁ keine Alkylgruppe oder durch eine Cycloalkylgruppe substituierte Alkylgruppe darstellt,
wobei es sich versteht, daß:
- die Begriffe "Alkyl" und "Alkoxy" für geradkettige oder verzweigte Gruppen, die 1 bis 6 Kohlenstoffatome enthalten, stehen,
- die Begriffe "Alkenyl" und "Alkinyl" für geradkettige oder verzweigte ungesättigte Gruppen, die 2 bis 6 Kohlenstoffatome enthalten, stehen,
- der Begriff "Cycloalkyl" für eine Gruppe mit 3 bis 8 Kohlenstoffatomen steht,
- der Begriff "Aryl" für eine Phenyl-, Naphthyl- oder Pyridylgruppe steht,
- der Begriff "substituiert" unter Bezugnahme auf die Begriffe "Aryl" und "Arylalkyl" bedeutet, daß diese Gruppen durch einen oder mehrere Reste ausgewählt aus Halogen, Alkyl, Alkoxy, Hydroxy und Alkyl substituiert durch ein oder mehrere Halogene, substituiert sein können;
deren Enantiomere und Diastereoisomere,
und deren Additionssalze mit einer pharmazeutisch annehmbaren Säure oder Base.

2. Verbindung der Formel (I) nach Anspruch 1, nämlich N-Methyl-2-(7-methoxynaphth-1-yl)-acetamid.

3. Verbindung der Formel (I) nach Anspruch 1, nämlich N-Methyl-3-(7-methoxynaphth-1-yl)-propionamid.

4. Verbindung der Formel (I) nach Anspruch 1, nämlich N-Propyl-4-(7-methoxynahth-1-yl)-butyramid.

5. Verfahren zur Herstellung der Verbindungen der Formel (I) nach Anspruch 1, dadurch gekennzeichnet, daß man eine Säure der Formel (II):
A - B - COOH (II)
in der A und B die in Anspruch 1 angegebenen Bedeutungen besitzen, mit einem Amin der Formel (III):
H₂N - R₁ (III)
in der R₁ die in Anspruch 1 angegebenen Bedeutungen besitzt, umsetzt zur Bildung der Verbindung der Formel (I/a): 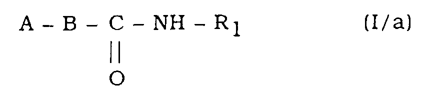 in der A, B und R₁ die oben angegebenen Bedeutungen besitzen,
welche Verbindung der Formel (I/a) durch Behandeln mit einem Thionierungsreagens die Verbindung der Formel (I/b) liefert: 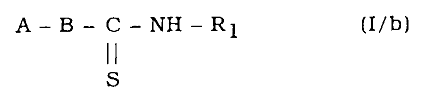 in der A, B und R₁ die oben angegebenen Bedeutungen besitzen,
welche Verbindungen der Formeln (I/a) und (I/b) die Gesamtheit der Verbindungen der Formel (I) nach Anspruch 1 bilden, welche gewünschtenfalls
- mit Hilfe einer oder mehrerer Reinigungsmethode ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können,
- und/oder mit einer pharmazeutisch annehmbaren Säure oder Base in die Salze überführt werden können.

6. Verfahren zur Herstellung der Verbindungen der Formel (I/c), einem Sonderfall der Verbindungen der Formel (I) nach Anspruch 1: 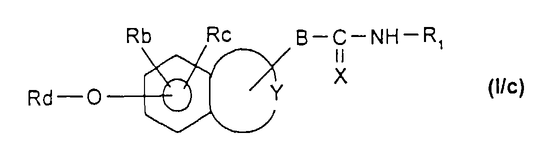 in der B, X, Y, R₁, R_{b}, R_{c} und R_{d} die in Anspruch 1 angegebenen Bedeutungen besitzen, dadurch gekennzeichnet, daß man die Gruppe R_{d} auf eine Verbindung der Formel (I/d) aufpfropft: 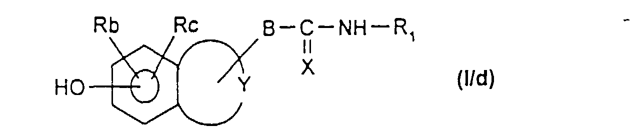 in der B, X, Y, R₁, R_{b} und R_{c} die oben angegebenen Bedeutungen besitzen, welche Verbindungen der Formel (I/c) gewünschtenfalls
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können
- und/oder mit einer pharmazeutisch annehmbaren Base in ihre Salze überführt werden können.

7. Verfahren zur Herstellung der Verbindungen der Formel (I/j): 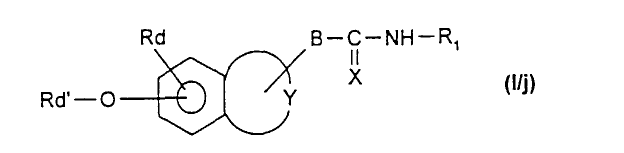 in der Rd, Y, X, B und R₁ die in Anspruch 1 angegebenen Bedeutungen besitzen und Rd' die gleichen Bedeutungen besitzen, wie sie oben für Rd definiert worden sind, dadurch gekennzeichnet, daß man die Gruppe Rd, wie sie oben definiert worden ist, auf die Hydroxyfunktion einer Verbindung der Formel (I/k) aufpfropft: 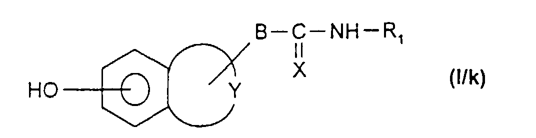 in der Y, B, X und R₁ die oben angegebenen Bedeutungen besitzen, so daß man eine Verbindung der Formel (I/*l*) erhält: 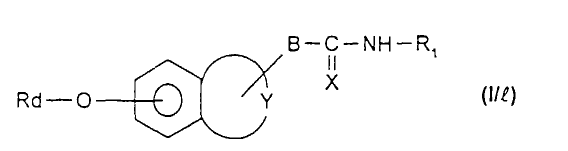 in der Rd, Y, B, X und R₁ die oben angegebenen Bedeutungen besitzen,
welche Verbindung der Formel (I/*l*) anschließend einer Umlagerungsreaktion unterworfen wird zur Bildung der Verbindung der Formel (I/m): 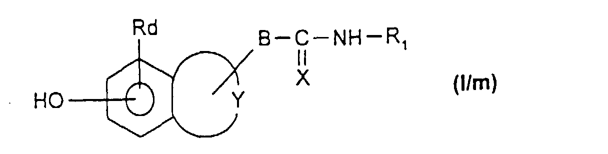 in der Rd, Y, B, X und R₁ die oben angegebenen Bedeutungen besitzen,
auf welche Hydroxygruppe erneut eine Gruppe Rd' aufgepfropft wird, wie sie oben definiert worden ist, zur Bildung einer Verbindung der Formel (I/j): 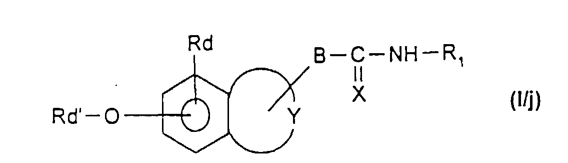 in der Rd, Rd', Y, B, X und R₁ die oben angegebenen Bedeutungen besitzen, welche Verbindungen der Formel (I/j) gewünschtenfalls
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung getrennt werden können
- und/oder mit einer pharmazeutisch annehmbaren Base in ihre Salze überführt werden können.

8. Pharmazeutische Zubereitungen enthaltend eine Verbindung der Formel (I) nach Anspruch 1 in Kombination mit einem oder mehreren pharmazeutisch annehmbaren Trägermaterialien.

9. Pharmazeutische Zubereitungen nach Anspruch 8 zur Behandlung von Störungen des melatoninergischen Systems und von Störungen, die mit dem melatoninergischen System verknüpft sind.

10. Zubereitungen nach Anspruch 8 oder 9 zur Behandlung von jahreszeitlich bedingten Depressionen, Schlafstörungen, kardiovaskulären pathologischen Zuständen, der Schlaflosigkeit und Müdigkeit als Folge von Zeitverschiebungen, Appetitstörungen und Fettsucht.

11. Verwendung der Verbindungen der Formel (I)_{A}: 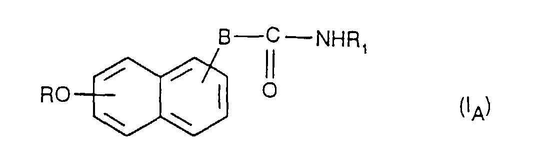 in der:
- R ein Wasserstoffatom oder eine geradkettige oder verzweigte (C₁-C₆)-Alkylgruppe,
- B eine Alkylenkette mit 2 bis 6 Kohlenstoffatomen und
- R₁ eine geradkettige oder verzweigte (C₁-C₆)-Alkylgruppe, die unsubstituiert oder durch eine oder mehrere (C₃-C₈)-Cycloalkylgruppen substituiert ist, bedeuten
von deren Enantiomeren und Diastereoisomeren
und von deren Additionssalzen mit einer pharmazeutisch annehmbaren Säure oder Base zur Herstellung von pharmazeutischen Zubereitungen zur BehandLung von Störungen des melatoninergischen Systems und von Störungen, die mit dem melatoninergischen System verknüpft sind.

12. Verwendung der Verbindungen der Formel (I_{A}): 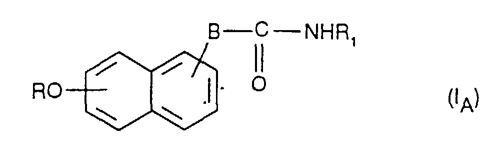 in der:
- R ein Wasserstoffatom oder eine geradkettige oder verzweigte (C₁-C₆)-Alkylgruppe,
- B eine Alkylenkette mit 2 bis 6 Kohlenstoffatomen und
- R₁ eine geradkettige oder verzweigte (C₁-C₆)-Alkylgruppe, die unsubstituiert oder durch eine oder mehrere (C₃-C₈)-Cycloalkylgruppen substituiert ist, bedeuten
von deren Enantiomeren und Diastereoisomeren
und von deren Additionssalzen mit einer pharmazeutisch annehmbaren Säure oder Base zur Herstellung von pharmazeutischen Zubereitungen zur Behandlung von jahreszeitlich bedingten Depressionen, Schlafstörungen, kardiovaskulären pathologischen Zuständen, Schlaflosigkeit und Müdigkeit als Folge von Zeitverschiebungen, Appetitstörungen und der Fettsucht.

## Claims

1. Compounds of formula (I) : 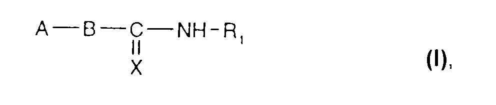 wherein :
- A represents a group of formula : 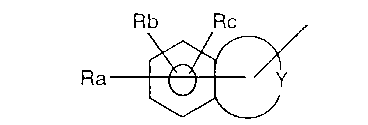
wherein :
- Y, together with the benzene ring carrying it, forms a ring system selected from naphthalene, dihydronaphthalene, tetrahydronaphthalene, benzofuran, 2,3-dihydrobenzofuran, benzothiophene, 2,3-dihydrobenzothiophene, indole, indoline, 2H-indene and indan ;
- Ra, Rb and Rc, each independently of the others, represents a hydrogen atom or a radical selected from halogen, hydroxy, -Rd and -O-Rd; with Rd being selected from alkyl, alkyl substituted by one or more halogen atoms, alkenyl, alkynyl, cycloalkyl, cycloalkylalkyl, aryl, arylalkyl, substituted aryl and substituted arylalkyl ;
it also being possible for Ra, together with Rb and the 2 adjacent carbon atoms carrying them, to form a ring system A₁ selected from furan, dihydrofuran, pyran and dihydropyran, A₁ being optionally substituted by one or more radicals or groups selected from hydroxy, alkyl, alkoxy and oxo ;
- B represents an alkylene chain having from 2 to 6 carbon atoms that is unsubstituted or substituted by one or more radicals selected from alkyl, alkoxy, hydroxy, carboxy, alkoxycarbonyl, carboxyalkyl and alkoxycarbonylalkyl, it being understood that B may also represent a methylene chain when Y, together with the benzene ring carrying it, forms a naphthalene group, a dihydronaphthalene group or a tetrahydronaphthalene group and when Ra, Rb and Rc do not simultaneously represent hydrogen atoms ;
with the proviso that Ra, Rb and Rc cannot simultaneously represent hydrogen atoms or one hydrogen atom and 2 methyl radicals or 2 hydrogen atoms and one methyl radical when Y, together with the benzene ring to which it is bonded, forms an indole group,
and that the compound of formula (I) cannot be N-methyl-3-(4-methoxy-naphth-1-yl)propionamide or N-tert-butyl-3-carboxy-4-(naphth-1-yl)butyramide ;
- X represents an oxygen atom or a sulphur atom ;
- and R₁ represents a radical selected from alkyl, alkyl substituted by one or more halogen atoms, alkenyl, alkynyl, cycloalkyl and cycloalkylalkyl ;
it being understood that :
when Y, together with the benzene ring carrying it, forms a naphthalene ring system, B represents an alkylene chain having from 2 to 6 carbon atoms, X represents an oxygen atom, 2 of the groups Ra, Rb or Rc simultaneously represent a hydrogen atom and the third represents a hydroxy group or a linear or branched (C₁-C₆)alkoxy group (Ra is in this case a substituent of the benzo moiety),
then R₁ cannot represent an alkyl group or an alkyl group substituted by a cycloalkyl group,
it being understood that :
- the terms "alkyl" and "alkoxy" denote linear or branched groups containing from 1 to 6 carbon atoms,
- the terms "alkenyl" and "alkynyl" denote unsaturated, linear or branched groups containing from 2 to 6 carbon atoms,
- the term "cycloalkyl" denotes a group having from 3 to 8 carbon atoms,
- the term "aryl" denotes a phenyl, naphthyl or pyridyl radical,
- the term "substituted" applied to the expressions "aryl" and "arylalkyl" indicates that such groups may be substituted by one or more radicals selected from halogen, alkyl, alkoxy, hydroxy and alkyl substituted by one or more halogen atoms ;
enantiomers and diastereoisomers thereof,
and addition salts thereof with a pharmaceutically acceptable acid or base.

2. Compound of formula (I) according to claim 1 which is N-methyl-2-(7-methoxynaphth-1-yl)acetamide.

3. Compound of formula (I) according to claim 1 which is N-methyl-3-(7-methoxynaphth-1-yl)propionamide.

4. Compound of formula (I) according to claim 1 which is N-propyl-4-(7-methoxynaphth-1-yl)butyramide.

5. Process for the preparation of compounds of formula (I) according to claim 1, characterised in that an acid of formula (II) :
A―B―COOH **(II),**
wherein A and B are as defined in claim 1,
is reacted with an amine of formula (III) :
H₂N―R₁ **(III),**
wherein R₁ is as defined in claim 1,
to obtain the compound of formula (I/a) ; 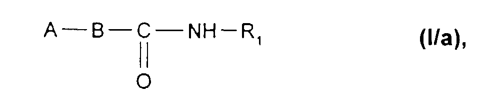 wherein A, B and R₁ are as defined hereinbefore,
which compound of formula (I/a), on treatment with a thionating reagent, yields the compound of formula (I/b) : 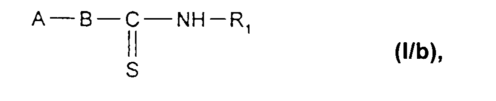 wherein A, B and R₁ are as defined hereinbefore,
the compounds of formulae (I/a) and (I/b) constituting the totality of compounds of formula (I) according to claim 1, which may be, if desired,
- purified according to one or more purification methods selected from crystallisation, chromatography, extraction, filtration, and passage over carbon or resin,
- separated, where appropriate, in pure form or in the form of a mixture, into their possible enantiomers or diastereoisomers,
- and/or converted into salts with a pharmaceutically acceptable acid or base.

6. Process for the preparation of compounds of formula (I/c), a particular case of the compounds of formula (I) according to claim 1 : 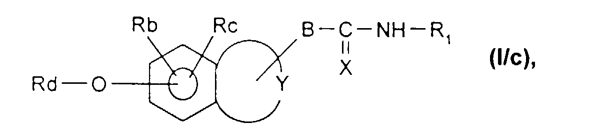 wherein B, X, Y, R₁, R_{b}, R_{c} and R_{d} are as defined in claim 1,
characterised in that the radical R_{d} is grafted onto a compound of formula (I/d) : 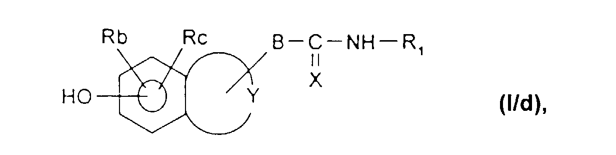 wherein B, X, Y, R₁, R_{b} and R_{c} are as defined hereinbefore,
which compounds of formula (I/c) may be, if desired,
- purified according to one or more purification methods selected from crystallisation, chromatography, extraction, filtration, and passage over carbon or resin,
- separated, where appropriate, in pure form or in the form of a mixture, into their possible enantiomers or diastereoisomers,
- and/or converted into salts with a pharmaceutically acceptable acid or base.

7. Process for the preparation of compounds of formula (I/j) : 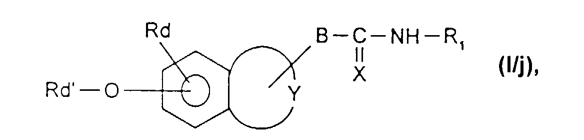 wherein Rd, Y, X, B and R₁ are as defined in claim 1 and Rd' may have the same meanings as Rd as defined hereinbefore,
characterised in that the radical Rd as defined hereinbefore is grafted onto the hydroxy function of a compound of formula (I/k) : 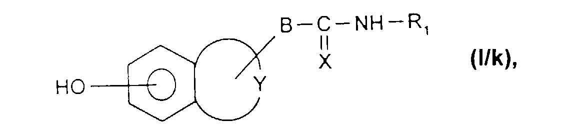 wherein Y, B, X and R₁ are as defined hereinbefore, to obtain a compound of formula (I/ℓ), 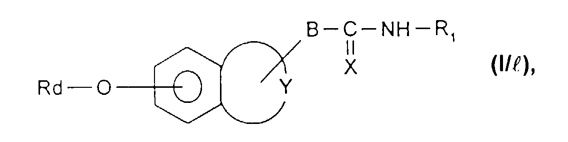 wherein Rd, Y, B, X and R₁ are as defined hereinbefore,
which compound of formula (I/ℓ) is then subjected to a rearrangement reaction to obtain the compound of formula (I/m) : 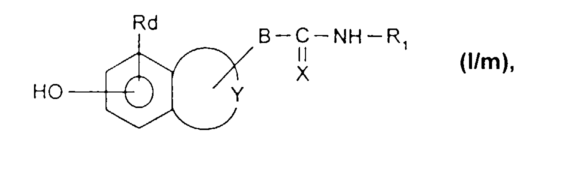 wherein Rd, Y, B, X and R₁ are as defined hereinbefore,
a radical Rd' as defined hereinbefore being again grafted onto the hydroxy function to obtain a compound of formula (I/j) : 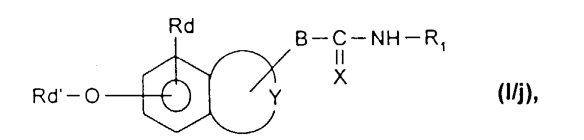 wherein Rd, Rd', Y, B, X and R₁ are as defined hereinbefore,
which compounds of formula (I/j) may be, if desired,
- purified according to one or more purification methods selected from crystallisation, chromatography, extraction, filtration, and passage over carbon or resin,
- separated, where appropriate, in pure form or in the form of a mixture, into their possible enantiomers or diastereoisomers,
- and/or converted into salts with a pharmaceutically acceptable acid or base.

8. Pharmaceutical compositions comprising a compound of formula (I) according to claim 1 in combination with one or more pharmaceutically acceptable excipients.

9. Pharmaceutical compositions according to claim 8 for use in the treatment of disorders of the melatoninergic system and disorders associated with the melatoninergic system.

10. Compositions according to claim 8 or claim 9 for use in the treatment of seasonal affective disorder, sleep disorders, cardiovascular pathologies, insomnia and fatigue due to jetlag, appetite disorders and obesity.

11. Use, for obtaining pharmaceutical compositions intended for the treatment of disorders of the melatoninergic system and disorders associated with the melatoninergic system, of compounds of formula (I_{A}): 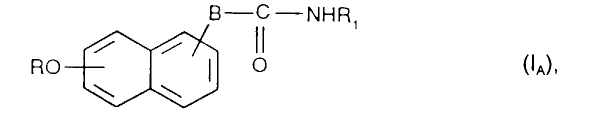 wherein :
- R represents a hydrogen atom or a linear or branched (C₁-C₆)alkyl group,
- B represents an alkylene chain having from 2 to 6 carbon atoms,
- R₁ represents a linear or branched (C₁-C₆)alkyl group unsubstituted or substituted by one or more (C₃-C₈)cycloalkyl groups,
enantiomers and diastereoisomers thereof,
and addition salts thereof with a pharmaceutically acceptable acid or base.

12. Use, for obtaining pharmaceutical compositions intended for the treatment of seasonal affective disorder, sleep disorders, cardiovascular pathologies, insomnia and fatigue due to jetlag, appetite disorders and obesity, of compounds of formula (I_{A}): 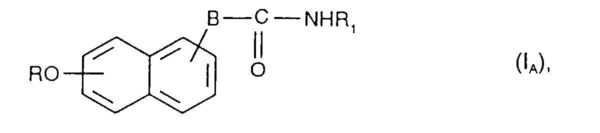 wherein :
- R represents a hydrogen atom or a linear or branched (C₁-C₆)alkyl group,
- B represents an alkylene chain having from 2 to 6 carbon atoms,
- R₁ represents a linear or branched (C₁-C₆)alkyl group unsubstituted or substituted by one or more (C₃-C₈)cycloalkyl groups,
enantiomers and diastereoisomers thereof,
and addition salts thereof with a pharmaceutically acceptable acid or base.
